# EUROPEAN PATENT SPECIFICATION

(11) **EP 0 688 224 B1**
(45) Date of publication and mention of the grant of the patent: **27.09.2000**
(21) Application number: 94909837.0
(22) Date of filing: 24.02.1994
(51) Int. Cl.: C12N 15/09, C12N 15/11, C12N 15/12, C12N 15/63, C12N 15/70, C12N 15/79, C07K 14/00, C07H 21/04

(54) **A MUTANT CRP-PROTEIN AND METHODS AND MATERIALS FOR MAKING AND USING IT**
EIN MUTIERTES CRP-PROTEIN UND VERFAHREN UND MITTEL ZU DESSEN HERSTELLUNG UND VERWENDUNG
PROTEINE CRP MUTANTE, PROCEDES ET MOYENS POUR SA FABRICATION ET SON UTILISATION

(30) Priority: 26.02.1993 US 23952
(43) Date of publication of application: 27.12.1995
(73) Proprietor: IMMTECH INTERNATIONAL INCORPORATED, Vernon Hills, IL 60061 (US)
(72) Inventor: POTEMPA, Lawrence A., Deerfield, IL 60015 (US); LIAO, Hans H., Madison, WI 53717 (US)
(74) Representative: Allard, Susan Joyce
(86) International application number: US9402181
(87) International publication number: WO9418999

(56) References cited:
- WO-A-89/09628
- INFLAMMATION, vol. 16, 1992, pages 93-9, XP000655098 M. L. TENCHINI ET AL: "Comparison of Sequence of cDNA Clone with Other Genomic and cDNA Sequences for Human C- Reactive Protein"
- IMMUNOLOGIC RESEARCH, vol. 10, 1991, pages 43-53, XP000655001 J. M. KILPATRICK AND J. E. VOLANAKIS: "Molecular Genetics, Structure, and Function of C-Reactive Protein"
- MOLECULAR IMMUNOLOGY, vol. 24, no. 5, 1987, pages 531-41, XP000654990 L. A. POTEMPA ET AL: "Expression, Detection and Assay of a Neoantigen (Neo-CRP) Associated with a Free, Human C-Reactive Protein Subunit"
- Journal of Biological Chemistry, Volume 261, No. 22, issued 05 August 1986, N.Y. NGUYEN et al., "The Amino Acid Sequence of Limulus C-Reactive Protein", pages 10456-10465, see entire document.
- Journal of Biological Chemistry, Volume 267, No. 35, issued 15 December 1992, A. AGRAWAL et al., "Probing the Phosphocholine-Binding Site of Human C-Reactive Protein by Site-Directed Mutagenesis", pages 25352-25358, see entire document.

## Description

### FIELD OF THE INVENTION

This invention relates to a mutant protein having at least one of the biological activities of modified-C-reactive protein and to methods and materials for making the mutant protein by recombinant DNA techniques. The invention also relates to methods and materials for using the mutant protein.

### BACKGROUND OF THE INVENTION

C-reactive protein was first described by Tillett and Francis [J. Exp. Med., 52, 561-71 (1930)] who observed that sera from acutely ill patients precipitated with the C-polysaccharide of the cell wall of Streptococcus pneumoniae. Others subsequently identified the reactive serum factor as protein, hence the designation "C-reactive protein."

C-reactive protein (CRP) is synthesized in the liver, and its concentration in serum may increase as much as 1,000-fold during the acute phase response. See Gewurz et al., Adv. Int. Med., 27, 345-372 (1982); Kushner, Ann. N.Y. Acad. Sci., 389, 39-48 (1982); Pepys et al., Adv. Immunol., 34, 141-212 (1983). Although the exact role of CRP in the acute phase response is not known, it is believed to play an important part in host defense. For instance, it has been reported that: (1) CRP binds phosphorylcholine, suggesting a role for CRP as an opsonin for microorganisms and damaged tissue that have exposed phosphorylcholine groups; (2) CRP binds chromatin, suggesting that CRP may act to scavenge chromatin released by cell lysis; (3) CRP neutralizes platelet activating factor, suggesting that CRP may function as a regulator of platelet and neutrophil activities; and (4) CRP complexed to certain other molecules or liposomes activates complement, suggesting that CRP may trigger the complement cascade. See Kaplan et al., J. Immunol., 112, 2135-2147 (1974); Volanakis et al., J. Immunol., 113, 9-17 (1974); Siegel et al., J. Exp. Med., 140, 631-47 (1974); Siegel et al., J. Exp. Med., 142, 709-21 (1975); Mold et al., J. Exp. Med., 154, 1703-1708 (1981); Narkates et al., Proc. N.Y. Acad. Sci., 389, 172-182 (1982); Nakayama et al., Clin. Exp. Immunol., 54, 319-326 (1983); Robey et al., J. Biol. Chem., 259, 7311-7316 (1984); Robey et al., J. Exp. Med., 161, 1344-56 (1985); Vigo, J. Biol. Chem., 260, 3418-3422 (1985); Shephard et al., Clin. Exp. Immunol., 63, 718-27 (1986); Horowitz et al., J. Immunol., 138, 2598-2603 (1987); Tatsumi et al., Clinica Chimica Acta, 172, 85-92 (1988); DuClos et al., J. Immunol., 141, 4266-4260 (1988); DuClos et al., J. Immunol., 146, 1220-1225 (1991); Xia et al., FASEB J., 6, 1344a (1992).

CRP is a pentamer which consists of five identical subunits, each having a molecular weight of about 23,500. The pentameric form of CRP is sometimes referred to as "native CRP."

In about 1983, another form of CRP was discovered which is referred to as "modified-CRP" or "mCRP". Modified-CRP has significantly different charge, size, solubility and antigenicity characteristics as compared to native CRP. Potempa et al., Mol. Immunol., 20, 1165-75 (1983). Modified-CRP also differs from native CRP in binding characteristics; for instance, mCRP does not bind phosphorylcholine. Id.; Chudwin et al., J. Allergy Clin. Immunol., 77, 216a (1986). Finally, mCRP differs from native CRP in its biological activity. See Potempa et al., Protides Biol. Fluids, 34, 287-290 (1986); Potempa et al., Inflammation, 12, 391-405 (1988).

The distinctive antigenicity of mCRP has been referred to as "neo-CRP." Neo-CRP antigenicity is expressed on:
1) denatured CRP prepared using suitable conditions (described below);
2) the primary translation product of DNA coding for CRP (preCRP); and
3) CRP immobilized on solid surfaces. Potempa et al., Mol. Immunol., 20, 1165-75 (1983); Mantzouranis et al., Ped. Res., 18, 260a (1984); Samols et al., Biochem. J., 227, 759-65 (1985); Chudwin et al., J. Allergy Clin. Immunol., 77, 216a (1986); Potempa et al., Inflammation, 12, 391-405 (1988).

The neo-CRP antigenicity may be detected with antibodies. For instance, an antiserum made specific for neo-CRP can be used. See Potempa et al., Mol. Immunol., 24, 531-41 (1987). Alternatively, the unique antigenic determinants of mCRP can be detected with monoclonal antibodies. Suitable monoclonal antibodies are described in published PCT application WO 91/00872, published January 24, 1991 and in Ying et al., J. Immunol., 143, 221-228 (1989), Ying et al., Immunol., 76, 324-330 (1992), and Ying et al., Molec. Immunol., 29, 677-687 (1992).

A molecule reactive with antiserum specific for neo-CRP has been identified on the surface of 10-25% of peripheral blood lymphocytes (predominantly NK and B cells), 80% of monocytes and 60% of neutrophils, and at sites of tissue injury. Potempa et al., FASEB J., 2, 731a (1988); Bray et al., Clin. Immunol. Newsletter, 8, 137-140 (1987); Rees et al., Fed. Proc., 45, 263a (1986). In addition, it has been reported that mCRP can influence the development of monocyte cytotoxicity, improve the accessory cell function of monocytes, potentiate aggregated-IgG-induced phagocytic cell oxidative metabolism, and increase the production of interleukin-1, prostaglandin E and lipoxygenase products by monocytes. Potempa et al., Protides Biol. Fluids, 34, 287-290 (1987); Chu et al., Proc. Amer. Acad. Cancer Res., 28, 344a (1987); Potempa et al., Proc. Amer. Acad. Cancer Res., 28, 344a (1987); Zeller et al., Fed. Proc., 46, 1033a (1987); Potempa et al., Inflammation, 12, 391-405 (1988); Chu et al., Proc. Amer. Acad. Cancer Res., 29, 371a (1988). Chudwin et al., J. Allergy Clin. Immunol., 77, 216a (1986) teaches that mCRP can have a protective effect in mice challenged with gram-positive type 7F Streptococcus pneumoniae.

Other activities of mCRP have been discovered and are described in certain published PCT applications. In particular, it has been discovered that mCRP binds immune complexes and aggregated immunoglobulin and can, therefore, be used to remove immune complexes and aggregated immunoglobulin from fluids and to quantitate immune complexes. See published PCT application WO 89/09628, published October 19, 1989. Modified-CRP has also been found to be effective in treating viral infections (see PCT application WO 93/10799, non-Streptococcal bacterial infections and endotoxic shock (see PCT application WO 93/10800, and cancer (see PCT application WO 93/21944.

For a brief review of CRP and mCRP, see Gotschlich, Ann. N.Y. Acad. Sci., 557, 9-18 (1989). Kilpatrick and Volanakis, Immunol. Res., 10, 43-53 (1991) provides a recent review of CRP.

Prior to the present invention, mCRP was preferably made using purified CRP as a starting material. Generally, mCRP was prepared from CRP by denaturing the CRP. For instance, CRP could be denatured by: (1) treatment with an effective amount of urea (preferably 8M) in the presence of a conventional chelator (preferably ethylenediamine tetraacetic acid (EDTA) or citric acid); (2) adjusting the pH of the CRP to below about 3 or above about 11-12; or (3) heating CRP above 50°C for a time sufficient to cause denaturation (preferably at 63°C for 2 minutes) in the absence of calcium or in the presence of a chelator such as those listed above. Urea treatment has been the preferred method. In addition, mCRP can be prepared from CRP by adsorbing the CRP onto solid surfaces. It is believed that mCRP prepared from CRP is formed by the dissociation of the five CRP subunits, each of which then undergoes a spontaneous conformational change to form mCRP. See Bray et al., Clin. Immunol. Newsletter, 8, 137-140 (1987).

Although biological sources of CRP and methods of purifying it from those sources are well known, purified CRP can be obtained from such sources only in limited quantities. Accordingly, mCRP could not be produced from CRP in commercial quantities.

Genomic and cDNA clones coding for human, mouse, and rabbit CRP have been isolated. Tucci et al., J. Immunol., 131, 2416-2419 (1983); Whitehead et al., Science, 221, 69-71 (1983); Lei et al., J. Biol. Chem., 260, 13377-83 (1985); Woo et al., J. Biol. Chem., 260, 13384-88 (1985); Hu et al., Biochem., 25, 7834-39 (1986); Samols and Hu, Protides Biol. Fluids, 34, 263-66 (1986); Syin et al., J. Biol. Chem., 261, 5473-79 (1986); Ciliberto et al., Nucleic Acids Res., 15, 5895 (1987); Hu et al., J. Biol. Chem., 263, 1500-1504 (1988); Whitehead et al., Biochem. J., 266, 283-90 (1990). To obtain pentameric native CRP, eukaryotic host cells, preferably mammalian host cells, should be used. See Samols and Hu, Protides Biol. Fluids, 34, 263-66 (1986); Hu et al., J. Biol. Chem., 263, 1500-1504 (1988). Thus, native CRP could be produced in large quantities by recombinant DNA techniques and then converted into mCRP as described above. However, it would be convenient to have a direct method of making mCRP or a molecule having the biological activities of mCRP by recombinant DNA techniques.

As noted above, the primary translation product of the CRP mRNA (preCRP) has been found to express neo-CRP antigenicity. Accordingly, mCRP could be prepared by selecting conditions so that the CRP subunits are not assembled into pentameric native CRP in the host cell. This can be accomplished by expressing a CRP genomic or cDNA clone in a prokaryotic host. See Samols and Hu, Prot. Biol. Fluids, 34, 263-66 (1986).

In attempting to produce mCRP in this manner, Applicants have discovered that the product of a CRP cDNA clone expressed in Escherichia coli consists of aggregates of CRP subunits and/or preCRP and CRP fragments, as well as free CRP subunits and/or preCRP. This cDNA product is extremely insoluble, and purification has proved problematical. In particular, Applicants have discovered that a substantial proportion of the aggregates in these preparations are formed by covalent cross-linking, and such cross-linked aggregates must be discarded, thereby significantly reducing yields.

Therefore, being able to produce a mutant CRP ,subunit or preCRP molecule having the biological activities of mCRP, but which was less likely to form covalently cross-linked aggregates than the unmutated protein, would be highly desirable in order to make processing and purification easier and more efficient. The present invention provides mutant proteins having these characteristics, and these mutant proteins may be produced by site-directed mutagenesis of a CRP cDNA or genomic clone as further described below.

Agrawal et al., FASEB J., 6, 1427a (1992) reports the use of site-specific mutagenesis of a CRP cDNA clone to investigate the structural determinants of the phosphorylcholine binding site of CRP. Eight mutant recombinant CRP's were prepared: Tyr40-->Phe; Glu42-->Gln; Tyr40-->Phe and Glu42-->Gln; Lys57-->Gln; Arg58-->Gly; Lys57-->Gln and Arg58-->Gly; Trp67-->Lys; and Lys57-->Gln, Arg58-->Gly and Trp67-->Lys. The authors concluded that Trp67 is critical for the structure of the phosphorylcholine binding site of CRP, that Lys57 and Arg58 also participate in the formation of this binding site, and that the tetrapeptide 39-Phe-Tyr-Thr-Glu has only a minimal or no role in the formation of this binding site.

### SUMMARY OF THE INVENTION

The invention provides a mutant protein which has the same amino acid sequence as an unmutated CRP subunit or an unmutated preCRP, except that at least one amino acid of the unmutated CRP subunit or unmutated preCRP has been deleted, at least one amino acid of the unmutated CRP subunit or unmutated preCRP has been replaced by another amino acid, at least one amino acid has been added to the unmutated CRP subunit or unmutated preCRP, or a combination of such changes has been made. The amino acid(s) added, deleted and/or replaced are chosen so that the mutant protein is less likely to form covalently cross-linked aggregates than the unmutated CRP subunit or unmutated preCRP. The mutant protein also exhibits at least one of the biological activities of mCRP.

The invention further provides a DNA molecule coding for the mutant protein of the invention and a vector for expression of the mutant protein. The vector comprises a DNA sequence coding for a mutant protein of the invention operatively linked to expression control sequences.

There is also provided a host cell which has been transformed so that it contains DNA coding for a mutant protein of the invention. The DNA coding for the mutant protein is operatively linked to expression control sequences.

The invention further provides a method of producing the mutant protein of the invention. The method comprises culturing a host cell which has been transformed so that it contains DNA coding for a mutant protein. The DNA coding for the mutant protein is operatively linked to expression control sequences. The culturing takes place under conditions permitting expression of the mutant protein.

The invention also provides methods of using the mutant proteins of the invention. In particular, the mutant proteins of the invention will have at least one of the biological activities of mCRP and can be used as mCRP would be used. For instance, the mutant proteins of the invention bind aggregated immunoglobulin and immune complexes. They can, therefore, like mCRP, be used to remove aggregated immunoglobulin and immune complexes from fluids, to quantitate immune complexes, and to reduce the levels of immune complexes in a mammal in need thereof. The mutant proteins of the invention can also be used to treat viral infections, bacterial infections, endotoxic shock and cancer.

The invention further provides a device for removing aggregated immunoglobulin and immune complexes from fluids. The device comprises a solid surface to which is bound a mutant protein of the invention. The device also comprises a means for encasing the solid surface so that the fluid may be contacted with the solid surface.

The invention also provides a kit for quantitating immune complexes. The kit comprises a container of one of the mutant proteins of the invention.

Finally, the invention provides a therapeutic composition comprising a mutant protein of the invention in combination with a pharmaceutically-acceptable carrier and an imaging agent comprising a mutant protein of the invention which is labeled so as to allow for detection of immune complexes or cancer cells in a mammal.

### BRIEF DESCRIPTION OF THE DRAWINGS

Figure 1A is a diagram of a series of polymerase chain reactions used to produce a mutant protein of the invention.
Figure 1B is a restriction map of plasmid pIT4.
Figures 2A and 2B illustrate the preparation of plasmid pIT3.
Figures 3A-D are elution profiles of materials chromatographed on a Q-Sepharose Fast Flow^{R} (Pharmacia) column. Figure 3A is native CRP, Figure 3B is mCRP, Figure 3C is wild-type recombinant CRP, and Figure 3C is a mutant protein according to the invention.
Figure 4 is a PhastGel^{R} SDS-PAGE gel (Pharmacia) which has been stained with Coomassie blue.
Figures 5A-B are graphs of the results of ELISA assays to detect the presence of native CRP and mCRP antigenic determinants on wild-type recombinant CRP.
Figures 5C-D are graphs of the results of ELISA assays to detect the presence of mCRP antigenic determinants on wild-type recombinant CRP and a mutant protein according to the invention, both of which have been purified by passage over a Q-Sepharose Fast Flow^{R} column.
Figures 6A-B are graphs of the results of ELISA assays to detect binding to aggregated IgG and monomeric IgG.

### DETAILED DESCRIPTION OF THE PRESENTLY PREFERRED EMBODIMENTS

The mutant proteins of the invention have at least one amino acid added, deleted or replaced as compared to an unmutated CRP subunit or unmutated preCRP. However, the mutant proteins may have several amino acid changes as compared to the unmutated CRP subunit or unmutated preCRP. For instance, the mutant proteins may have several added amino acids, several deleted amino acids, several replacement amino acids, or a combination of added, deleted or replacement amino acids, as compared to the unmutated CRP subunit or preCRP.

The amino acid(s) added, deleted and/or replaced are chosen so that the mutant protein is less likely to form covalently cross-linked aggregates than the unmutated CRP subunit or unmutated preCRP. Suitable amino acid changes include the deletion or replacement of at least one, preferably all, of the cysteines in an unmutated CRP subunit or unmutated preCRP. CRP subunits contain two cysteines and preCRP's contain three cysteines, and it is believed that some of these cysteines form intermolecular disulfide bonds, thereby contributing to the formation of covalently cross-linked aggregates. Therefore, one or, preferably, both of these cysteines are desirably deleted or replaced. When the cysteines are replaced with other amino acids, they are preferably replaced with glycine, alanine, valine, leucine, isoleucine, serine, threonine or methionine, but any amino acid can be used. Most preferred is substitution with alanine.

Lysine and derivatized lysine residues may also contribute to intermolecular covalent cross-linking. Accordingly, suitable amino acid changes may also include the deletion or replacement of at least one of the lysines in an unmutated CRP subunit or unmutated preCRP.

As a result of the amino acid changes in them, the mutant proteins of the invention are easier to purify with much higher yields than unmutated CRP subunits or unmutated preCRP's. Also, the final product is much purer with many fewer aggregates and fragments than that obtained with unmutated CRP subunits or unmutated preCRP's (see, e.g., Example 1).

Not all of the amino acid additions, deletions and replacements need contribute to the reduced likelihood of forming covalently cross-linked aggregates as long as the combined effect of all the changes is a reduction in intermolecular covalent cross-linking. For instance, the recombinant DNA manipulations used to produce the mutant proteins may result in amino acids being added at the amino or carboxy terminal ends of the CRP subunit. This is acceptable as long as these amino acids do not contribute to the production of covalently cross-linked aggregates (see, e.g., Example 1). In addition, some of the amino acid changes may be made for other purposes. For instance, it is desirable to make amino acid changes which increase the solubility of the resultant mutant protein in aqueous media, since a more soluble mutant protein is easier to purify and process. Suitable amino acid changes to increase the solubility include deleting one or more hydrophobic amino acids, replacing one or more hydrophobic amino acids with charged amino acids, adding one or more charged amino acids, or combinations of these changes. However, for the reasons stated above, it may be desirable to avoid the addition of lysine residues. Aqueous media include water, saline, buffers, culture media, and body fluids.

The mutant proteins of the invention also exhibit at least one of the biological activities of mCRP. As used herein, "biological activity" refers to properties of mCRP other than its physical and chemical properties. The biological activities of mCRP include its ability to bind aggregated immunoglobulin and immune complexes which allows mCRP to be used to removed aggregated immunoglobulin and immune complexes from fluids (such as antibody reagents or body fluids), to quantitate immune complexes, and to reduce the levels of immune complexes in a mammal in need thereof. The biological activities of mCRP also include its effectiveness in treating viral infections, bacterial infections, endotoxic shock and cancer.

For instance, it has been found that the mutant proteins of the present invention can bind aggregated immunoglobulin and immune complexes. The binding of the aggregated immunoglobulin or immune complexes may be accomplished by adding a mutant protein directly to a fluid containing aggregated immunoglobulin or immune complexes, or a mutant protein may first be immobilized on a solid support before being contacted with the fluid containing the aggregated immunoglobulin or immune complexes. When the mutant protein is bound on a solid support, fluids may be incubated statically on the immobilized mutant protein when used for diagnostic assays, or fluids may be passed dynamically across the immobilized mutant protein in an extracorporeal device when used for therapeutic treatment to bind immune complexes in a body fluid.

Suitable solid support materials for use in the present invention may be made of agarose-based resin, polyacrylamide, polymethyl-methacrylate, polycarbonate, polysulfone, polyacrylonitrile, polyethylene, polypropylene, latex, dextran, glass, nylon, polyvinyl alcohol, gels, clay, cellulose derivatives, and any other hydrophobic or hydrophilic polymeric material. The solid support may be in the form of beads for use in a column, may be in the form of the wells of a microtiter plate, may be in the form of a hollow fiber membrane, or may take other forms as discussed below. Column and solid phase materials are commercially available in the United States from Bio Rad Laboratories (Richmond, CA); Pierce Chemical Co. (Rockford, IL); Pall Biosupport (Glen Cove, NY); Micro Membranes (Newark, NJ); Pharmacia Fine Chemicals (Uppsala, Sweden); and others.

The mutant protein may be immobilized by covalent or non-covalent binding to the solid support. Methods of immobilizing proteins on solid supports are well known in the art. For instance, mutant proteins may be immobilized on solid supports by simply incubating the mutant protein with the solid support to adsorb the mutant protein.

To ensure maximum binding of aggregated immunoglobulin or immune complexes to the mutant protein, a linking agent may be utilized to secure the attachment of the mutant protein to polymeric materials. The mutant protein may be immobilized non-covalently or covalently on the surface of the polymeric material with the linking agent. For purposes of this invention, linking agents are incorporated as part of, or derivatized onto, the polymeric solid surface before the mutant protein is added. The linking agents are conventional; they include diimidoesters, carbodiimide, periodate, alkylhalides, dimethylpimelimidate and dimaleimides [See Blait, A.H., and Ghose, T.I., J. Immunol. Methods, 59:129 (1983); Blair, A.H., and Ghose, T.I., Cancer Res., 41:2700 (1981); Gauthier, et al., J. Expr. Med., 156:766-777 (1982)].

Conjugation of the mutant protein to the linking agent generally requires an initial modification of protein amino acid R groups or cross linking agent or both. Such modifications may serve to selectively activate R groups (e.g., carbodiimide-O-acyl urea, intermediate formation with aspartic, glutamic, and C-terminal carboxyl residues) to allow for reaction with appropriate available agent functional groups (amino groups in the case of carbodiimide). Modifications can also include the introduction of a new reactive moiety (e.g., N-succinimidyl-3-(2-pyridyldithio)-propionate) which introduces pyridyldithio groups on lysine epsilon-amino residues. This allows for disulfide bond formation between protein and linking agent. In some cases, bifunctional coupling reagents are employed which form bridges between the protein R groups and the linking agent of interest.

The mutant proteins may be used to remove aggregated immunoglobulin or immune complexes from fluids used for research, in therapeutic procedures, or in diagnostic tests, e.g., solutions containing monoclonal antibodies, antisera, derivitized reagents, intravenous gamma globulin, or isolated blood components. The presence of aggregated immunoglobulin in such fluids may be expected because of processing steps used to make these fluids, such as heat treatment of antisera to inactivate complement. The mutant protein may be bound on a solid support for removing aggregated or complexed immunoglobulins from such fluids. Suitable solid supports are those described above, and the mutant protein is bound to them in the ways described above. Alternatively, the mutant protein may be added directly to such fluids in order to remove the aggregated or complexed immunoglobulins.

Mutant proteins of the present invention can also be used to remove immune complexes from body fluids, such as whole blood or plasma, by contacting the fluid with the mutant protein. The mutant protein may be added directly to the body fluid or may be immobilized on a solid support and then contacted with the body fluid. Suitable solid supports are those described above, and the mutant protein is bound to them in the ways described above.

Persistent high levels of immune complexes may be found in diseases such as cancer, autoimmune diseases, arthritis, and infections. The continued presence of immune complexes in the circulation and their deposition in tissues contributes to compromised immune system function and inflammatory pathology. Accordingly, reducing the level of immune complexes should be beneficial. See Theofilopoulos et al., Adv. Immunol., 28. 90-220 (1979); Theofilopoulos et al., Immunodiagnostics of Cancer, p. 896 (1979).

For such therapeutic uses, the mutant protein may be contacted with the body fluid by passing the blood, plasma or other fluid through an extracorporeal device having a solid support coated with mutant protein. The device also will have a means for encasing the solid support so that the fluid may contact the mutant protein bound to the solid support. The blood, plasma or other body fluid is circulated dynamically through the device so that the immune complexes contained therein are bound and removed as in, e.g., conventional plasmapheresis or hemodialysis techniques. The fluids can be returned to the body negating the need for blood replacement therapy.

The solid support and encasing means of the extracorporeal device may be made of any biocompatible material. For instance, the solid support may be a membranous surface, agarose-based beads or hollow fibers coated with mutant protein. The extracorporeal device may be a column packed with beads or a cylinder encasing a hollow fiber membrane. The device may also include appropriate tubing for connecting it to a patient and a pump to aid the passage of the fluid through the device and back into the patient and to prevent air from entering the system. In particular, conventional plasmapheresis devices may be used in the practice of the present invention by modifying them so that they contain a solid support on which mutant protein is immobilized. See, e.g., Randerson et al., Art. Organs, 6, 43-49 (1982); Smith et al., Cleve. Clin. Q., 51, 135-142 (1984); Nilsson et al., Plasma Ther. Transfus. Technol., 5, 127-134 (1984); Nilsson et al., in Affinity Adsorption of Inhibitors, pages 223-241 (Hoyer ed. 1984); Liberti, "Development Of A Universal Immune Specific Filtration Device (Blood Filter)," presented at Opportunities In The Oncology Marketplace; Mittelman et al., Seminars in Hematology, 26, 15-18 (1989); U.S. Patent No. 4,432,871, issued February 21, 1984; U.S. Patent No. 4,551,435, issued November 5, 1985; U.S. Patent No. 4,614,513, issued September 30, 1986 which describe conventional plasmapheresis devices.

The device must be sterilized for therapeutic use. Sterilization may be accomplished in conventional ways such as steam, heat, purging with ethylene oxide or irradiation.

The invention also comprises a method of detecting or quantitating immune complexes comprising contacting the immune complexes with a mutant protein of the invention so that the immune complexes bind to the mutant protein. The mutant protein may be added directly to fluids containing the immune complexes to detect or quantitate immune complexes in the fluids. Alternatively, the mutant protein may be immobilized on a solid support before contacting it with fluids containing immune complexes. The mutant protein may also be added directly to cells or a tissue sample having immune complexes thereon, and labeled mutant protein may be injected into a mammal so that it localizes in areas of the mammal's body where immune complexes are found, such as areas of inflammation.

Suitable solid support materials are those described above, and mutant protein is immobilized on them in the ways described above. For diagnostic assays, suitable solid supports include those conventionally used for immunoassays. For instance, the solid support may be test tubes, the wells of a microtiter plate, latex beads, glass beads, other beads, filter paper, glass fiber filter paper, or dipsticks made of, e.g., polycarbonate, polysulfone or latex.

To detect or quantitate the immune complexes, labeled mutant protein can be used. The labels useful in the invention are those known in the art such as enzyme, fluorescent, bioluminescent, chemiluminescent, and radioactive labels and biotin.

Alternatively, the immune complexes can be detected or quantified using conventional immunoassay techniques by adding a labeled component that binds to the immune complexes. Suitable labeled components include conventional reagents used in immunoassays. For instance, labeled antibodies to the immunoglobulin or the antigen in the immune complexes could be used or labeled Protein A which binds to the Fc portion of immunoglobulins could be used. The labels used are those described above.

Suitable conventional immunoassay techniques include agglutination, radioimmunoassay, enzyme immunoassays and fluorescence assays. For instance, the mutant protein may be coated onto latex beads for use in agglutination assays or onto dipsticks for use in qualitative or semi-quantitative immunoassays. Enzymelinked immunosorbent assays (EIA) are preferred since they provide a means for sensitive quantitation of levels of immune complexes. In general, any immunoassay technique can be used that results in an observable change in properties.

The specific concentrations of reagents, the temperatures and times of incubations, as well as other assay conditions can be varied in whatever assay is employed to detect or quantitate immune complexes depending on such factors as the concentration of the immune complexes in the sample, the nature of the sample, and the like. Those skilled in the art will be able to determine operative and optimal assay conditions for each determination while employing routine experimentation. Since body fluids from mammals normally contain immune complexes, comparison of the levels of immune complexes in a test sample from a mammal will have to be made to the levels found in normals to identify levels of immune complexes indicative of a disease state.

A test kit for detecting or quantitating immune complexes is also part of the invention. The kit comprises a container holding a solution of mutant protein or mutant protein attached to a solid support. The solid supports are the types described above, and the mutant protein is attached as described above. Thus, the container could be a bottle holding a solution of mutant protein, a dipstick coated with mutant protein encased in a protective package, a bottle holding latex beads coated with mutant protein, or a microtiter plate, the wells of which are coated with mutant protein.

The mutant protein may be labeled if it is to be used for detecting or quantitating the immune complexes. Alternatively, the kit may further comprise a container holding a labeled component that allows for the detection or quantitiation of the immune complexes by binding to the immune complexes or to the mutant protein. Suitable labeled components were described above.

The mutant proteins of the invention can be administered to a mammal to reduce the level of immune complexes in the mammal. It is believed that, upon introduction of the mutant protein into a body fluid of the mammal containing the immune complexes, the soluble immune complexes will grow larger in physical size and precipitate (fall out of solution) or will otherwise be modified to enhance their removal by phagocytes. To reduce the level of immune complexes in a mammal, an effective amount of a mutant protein is administered to the mammal by injection, preferably intravenous injection.

The mutant proteins of the invention can also be used to treat viral infections. They can be used to treat any type of viral infection such as Retroviridae infections. The Retroviridae are a family of spherical enveloped RNA viruses comprising three sub-families: Oncovirinae, Spurmavirinae and Lentivirinae. Hull et al, Virology: Directory & Dictionary of Animal, Bacterial and Plant Viruses, page 191 (Stockton Press 1989). Replication starts with reverse transcription of virus RNA into DNA which becomes integrated into the chromosomal DNA of the host. Id. Endogenous oncoviruses occur widely among vertebrates and are associated with many diseases. Id. The lentiviruses include HIV-1 and SIV. Fauci, Science, 239, 617-622 (1988).

To treat viral infections in a mammal, an effective amount of a mutant protein is administered to the mammal. The mutant protein is preferably administered to the mammal before the infection becomes too serious. Most preferably, the mutant protein is administered at the first indication of a viral infection or prophylactically to those at risk of developing viral infections. For instance, the mutant protein may be administered prophylactically to hemophiliacs or surgical patients who may receive blood contaminated with a virus such as HIV-1 or hepatitis. Of course, a mutant protein can be administered to a mammal already suffering from a viral infection.

The mutant protein will generally be administered to the mammal suffering from a viral infection by injection (e.g., intravenous, intraperitoneal, subcutaneous, intramuscular). Preferably intravenous injection is used. The mutant protein may be injected in a fluid or may be encapsulated in liposomes. The mutant protein may also be applied topically to, e.g., a wound or other site of infection. Finally, it should be possible to administer the mutant protein by means of a spray to treat respiratory infections.

The mutant proteins of the invention may also be used to treat bacterial infections, especially gram-negative bacterial infections, and endotoxic shock. Endotoxins are the lipopolysaccharide components of the outer membranes of gram-negative bacteria that trigger many of the adverse systemic reactions and serious sequelae in sepsis and gram-negative bacteremia.

To treat a bacterial infection or endotoxic shock in a mammal, an effective amount of a mutant protein is administered to the mammal. The mutant protein is preferably administered to the mammal before the bacterial infection becomes too serious and septic shock or endotoxic shock has developed. Most preferably, the mutant protein is administered at the first indication of a bacterial infection or prophylactically to those at risk of developing bacterial infections. For instance, a mutant protein may be administered prophylactically to surgical patients or patients in intensive care who are at risk of developing bacterial infections. Of course, the mutant protein can be administered to a mammal already suffering from a bacterial infection or already suffering from septic shock or endotoxic shock.

The mutant protein will generally be administered to the mammal suffering from a bacterial infection or endotoxic shock by injection (e.g., intravenous, intraperitoneal, subcutaneous, intramuscular). It is preferably administered by intravenous injection. The mutant protein may be administered in a fluid or may be encapsulated in liposomes. The mutant protein may also be applied topically to, e.g., a wound or other site of infection, and it should be possible to administer the mutant protein by means of a spray to treat respiratory infections.

Finally, the mutant proteins of the invention can be used to treat cancer. It is contemplated that the mutant proteins of the invention can be used to treat a variety of cancers, including but not limited to, adenocarcinoma, lymphoma, fibrosarcoma, and leukemia, and to reduce metastasis. The mutant protein should be administered to the mammal when the presence of cancer cells in the mammal is first detected.

For the treatment of cancer, the mutant protein is preferably administered to the mammal by injection (e.g., intravenous, intraperitoneal, subcutaneous, intramuscular). The mutant protein may be administered in a fluid or encapsulated in liposomes.

It is understood by those skilled in the art that the dose of mutant protein that must be administered to a mammal for therapeutic treatment will vary depending on the mammal which will receive the mutant protein, the reason for administering the mutant protein (e.g., to reduce the level of immune complexes, to treat a viral infection, to treat a bacterial infection, to treat endotoxic shock, to treat cancer), the severity of the mammal's condition, the route of administration, and the identity of any other drugs being administered to the mammal. It is also understood that it may be necessary to give more than one dose of the mutant protein.

Effective dosages and schedules for administration of the mutant protein may be determined empirically, and making such determinations is within the skill of the art. A dose of from about 5 µg to about 150 mg of mutant protein per kg of body weight, preferably from about 100 µg to about 20 mg per kg, will be effective for reducing the level of immune complexes, treating a viral infection, treating a bacterial infection, treating endotoxic shock or treating cancer. For treating cancer, the dose is most preferably from about 2 mg to about 10 mg per kg. Generally, one dose is sufficient for treating a bacterial infection or endotoxic shock. Multiple doses will generally be necessary for reducing the level of immune complexes, treating viral infections and treating cancer. If multiple doses are needed, the interval between doses is preferably from about 1 day to about 7 days. For treating cancer, the multiple doses are most preferably administered from about 1 to about 3 days apart. Administration of mutant protein should be continued until the level of immune complexes has returned to normal or until health has been restored to the mammal.

The mutant protein is administered to the mammal in a pharmaceutically-acceptable carrier. Pharmaceutically-acceptable carriers are well known. For instance, suitable carriers for the administration of the mutant proteins of the invention include fluids such as water, saline and buffers. Except when it is used to reduce the level of immune complexes, the mutant protein may also be administered encapsulated in liposomes [see Deodhar et al., Cancer Research, 42, 5084-5088 (1982); Thombre et al., Cancer Immunol. Immunother., 16, 145-150 (1984); Barna et al., Cancer Research, 44, 305-310 (1984)]. For treatment of cancer, the mutant proteins are preferably administered encapsulated in liposomes. The term "liposome" refers to any sac-like or hollow vesicle-like structure which is capable of encapsulating a mutant protein and includes multilamellar vesicles, unilamellar vesicles, and red blood cell ghosts. Methods of preparing lipsomes and encapsulating molecules in them are well known in the art. Preferably, the liposomes containing mutant protein are unilamellar vesicles formed by extrusion which may be prepared as described by MacDonald et al., Biochim. Biophys. Acta, 1061:297-301 (1991). For topical application, when appropriate, the mutant protein may be incorporated into lotions, gels, cremes, etc., as is well known in the art. It is within the skill in the art to determine acceptable and optimum carriers and routes of administration.

The mutant proteins may be administered to the mammal alone or in combination with other drugs. For instance, the mutant proteins may be administered in combination with antibiotics when used to treat a bacterial infection or may be administered with other anti-cancer agents when used for treating cancer. Effective dosages and schedules for administering the mutant proteins and these other drugs together may be determined empirically as set forth above. It is expected that smaller amounts of mutant protein and anti-cancer agent (50% or less) may be employed when they are used in combination as compared to when they are used individually to treat cancer. It is also within the skill in the art to determine acceptable and optimum carriers and routes of administration for the mutant proteins and the other drugs when they are used in combination.

The other cancer agents which can be used in combination with the mutant proteins include cytotoxic chemotherapeutic compounds known in the art such as Thiotepa, Busulfan, Cyclophosphamide, Methotrexate, Cytarabine, Bleomycin, Cisplatin, Doxorubicin, Melphalan, Mercaptopurine, Vinblastin, and 5-Fluorouracil. Other suitable chemotherapeutic compounds are listed in Krakoff, CA-A Cancer Journal For Clinicians, 41:264-278 (1991). Typically, cytotoxic agents function in destroying cells and/or preventing their multiplication and are thus, useful in treating cancer. The other anti-cancer agent may also be an immunoadjuvant or cytokine. Immunoadjuvants and cytokines, also referred to as "biological response modifiers," are known in the art. Generally, such molecules are useful in stimulating or enhancing host defense mechanisms and are therefore useful in anti-cancer therapy. Examples of immunoadjuvants or cytokines that may be administered include interferon(s), colony stimulating factor (CSF), tumor necrosis factor (TNF), hormones such as steroids, and interleukins such as IL-1, IL-2, and IL-6.

The mutant proteins may also be used as imaging agents for detecting immune complexes or cancer cells in vivo. To do so, a labeled mutant protein will be injected into a mammal and will localize in areas where immune complexes have been deposited (such as areas of inflammation) or in areas where cancer cells are located. Alternatively, an unlabeled mutant protein can be injected. Then a labeled material which binds to the mutant protein is injected, and this labeled material binds to the mutant protein in areas where it has localized. Methods of making and using imaging agents and suitable labels for them are well-known in the art.

The mutant proteins of the invention can be prepared by expression of DNA coding for them in transformed host cells. DNA coding for a mutant protein according to the invention can be prepared by in vitro mutagenesis of a CRP genomic or cDNA clone or can be chemically synthesized.

As discussed in the Background section, genomic and cDNA clones coding for human, mouse, and rabbit CRP have been isolated. There is substantial homology between the amino acid sequences of CRP's from different species. For instance, there is from about 50 to about 80% sequence homology between CRP's from various mammalian species. Hu et al., Biochem., 25, 7834-39 (1986); Whitehead et al., Biochem. J., 266, 283-90 (1990); Kilpatrick and Volanakis, Immunol. Res., 10, 43-53 (1991). Given the substantial homology between CRP's from different species, probes can readily be prepared so that genomic and cDNA clones can be isolated which code for CRP's from other species. Methods of preparing such probes and isolating genomic and cDNA clones are well known. See, e.g., Lei et al., J. Biol. Chem., 260, 13377-83 (1985); Woo et al., J. Biol. Chem., 260, 13384-88 (1985); Hu et al., Biochem., 25, 7834-39 (1986); Hu et al., J. Biol. Chem., 263, 1500-1504 (1988); Whitehead et al., Biochem. J., 266, 283-90 (1990).

Using one of the known clones or a newly-isolated clone, DNA coding for a mutant protein according to the invention can be prepared using conventional and well known in vitro mutagenesis techniques. Particularly preferred is site-directed mutagenesis using polymerase chain reaction (PCR) amplification. See Example 1. The following references describe other site-directed mutagenesis techniques which can be used to produce DNA coding for a mutant protein of the invention: Current Protocols In Molecular Biology, Chapter 8, (Ansubel ed. 1987); Smith & Gilliam, Genetic Engineering Principles And Methods, 3, 1-32 (1981); Zoller & Smith, Nucleic Acids Res., 10, 6487-6500 (1982); Zoller et al., Methods Enzymol., 100, 468-500 (1983); Zoller & Smith, DNA, 3, 479-88 (1984); Brake et al., Proc. Natl. Acad. Sci. USA, 81, 4642-46 (1984); Bio/Technology, pages 636-39 (July 1984); Botstein et al., Science, 229, 1193 (1985); Kunkel et al., Methods Enzymol., 154, 367-82 (1987).

DNA coding for a mutant protein of the invention can also be prepared by chemical synthesis. Methods of chemically synthesizing DNA having a specific sequence are well-known in the art. Such procedures include the phosphoramidite method (see, e.g., Beaucage and Caruthers, Tetrahedron Letters, 22, 1859 (1981); Matteucci and Caruthers, Tetrahedron Letters, 21, 719 (1980); and Matteucci and Caruthers, J. Amer. Chem. Soc., 103, 3185 (1981)) and the phosphotriester approach (see, e.g., Ito et al., Nucleic Acids Res., 10, 1755-69 (1982)).

The invention also includes a vector which comprises a DNA sequence coding for a mutant protein according to the invention. The DNA coding sequence is operatively linked in the vector to appropriate expression control sequences. Methods of effecting this operative linking, either before or after the DNA coding for the mutant protein is inserted into the vector, are well known. Expression control sequences include promoters, activators, enhancers, operators, stop signals, cap signals, polyadenylation signals, and other signals involved with the control of transcription.

The vector must contain a promoter and a transcription termination signal, both operatively linked to the DNA sequence, i.e., the promoter is upstream of the DNA sequence and the termination signal is downstream from it. The promoter may be any DNA sequence that shows transcriptional activity in the host cell and may be derived from genes encoding homologous or heterologous proteins and either extracellular or intracellular proteins, such as amylase, glycoamylases, proteases, lipases, cellulases, and glycolytic enzymes. Also, a promoter recognized by T7 RNA polymerase may be used if the host is also engineered to contain the gene coding for T7 RNA polymerase. The promoter may contain upstream or downstream activator and enhancer sequences. An operator sequence may also be included downstream of the promoter, if desired.

However, the promoter need not be identical to any natural-occurring promoter. It may be composed of portions of various promoters or may be partially or totally synthetic. Guidance for the design of promoters is provided by studies of promoter structure such as that of Harley and Reynolds, Nucleic Acids Res., 15, 2343-61 (1987). Also, the location of the promoter relative to the transcription start may be optimized. See Roberts, et al., Proc. Natl Acad. Sci. USA, 76, 760-4 (1979).

Expression control sequences suitable for use in the invention are well known. They include those of the E. coli lac system, the E. coli trp system, the TAC system and the TRC system; the major operator and promoter regions of bacteriophage lambda; the control region of filamentous single-stranded DNA phages; the expression control sequences of other bacteria; promoters derived from genes coding for Saccharomyces cerevisiae TPI, ADH, PGK and alpha-factor; promoters derived from genes coding for the Aspergillus oryzae TAKA amylase and A. niger glycoamylase, neutral alpha-amylase and acid stable alpha-amylase; promoters derived from genes coding for Rhizomucor miehei aspartic proteinase and lipase; mouse mammary tumor promoter; SV40 promoter; the actin promoter; and other sequences known to control the expression of genes of prokaryotic cells, eukaryotic cells, their viruses, or combinations thereof.

The vector may be a self-replicating vector or an integrative vector. If the vector is self-replicating, it must contain one or more replication systems which allow it to replicate in the host cells. In particular, when the host is a yeast, the vector should contain the yeast 2u replication genes REP1-3 and origin of replication.

When the vector is a self-replicating vector, it is preferably a high copy number plasmid so that high levels of expression are obtained. As used herein, a "high copy number plasmid" is one which is present at about 100 copies or more per cell. Many suitable high copy number plasmids are known and include bacterial plasmids such as pUC and yeast plasmids such as pC.

Alternatively, an integrating vector may be used which allows the integration into the host cell's chromosome of the DNA coding for the mutant proteins. Although the copy number of the coding sequences in the host cells would be lower than when self-replicating vectors are used, transformants having sequences integrated into their chromosomes are generally quite stable.

The vector should further include one or more restriction enzyme sites for inserting DNA sequences into the vector, and preferably contains a DNA sequence coding for a selectable or identifiable phenotypic trait which is manifested when the vector is present in the host cell ("a selection marker"). Suitable selection markers are well known in the art.

Suitable vectors for use in the invention are well known. They include retroviral vectors, vaccinia vectors, pUC (such as pUC8 and pUC4K), pBR (such as pBR322 and pBR328), pTZ (such as pTZ18R), pUR (such as pUR288), phage lambda, YEp (such as YEp24) plasmids, and derivatives of these vectors.

DNA coding for a signal or signal-leader sequence may be located upstream of the DNA sequences encoding the mutant proteins. A signal or signal-leader sequence is an amino acid sequence at the amino terminus of a protein which allows the protein to which it is attached to be secreted from the cell in which it is produced. Suitable signal and signal-leader sequences are well known and include the CRP presequence (see Background section), yeast ∝-factor signal sequence (see U.S. Patent Nos. 4,546,082 and 4,870,008), the yeast BAR1 secretion system (see U.S. Patent No. 4,613,572), Kluyveromyces lactis signal-leader sequence, and the yeast invertase signal sequence (Stetler et al., Bio/Technology, 7:55-60 (1989), Smith et al., Science, 229:1219-1229 (1985)). Although secreted proteins are often easier to purify, expression levels are much lower than those that can be obtained in the absence of secretion.

Chemical synthesis of DNA coding and other sequences is preferable for several reasons. First, chemical synthesis is desirable because codons preferred by the host in which the DNA sequence will be expressed may be used to optimize expression of the mutant proteins. Not all of the codons need to be altered to obtain improved expression, but greater than 50%, most preferably at least about 80%, of the codons should be changed to host-preferred codons.

The codon preferences of many host cells, including E. coli, yeast, and other prokaryotes and eukaryotes, are known. See Maximizing Gene Expression, pages 225-85 (Reznikoff & Gold, eds., 1986). The codon preferences of other host cells can be deduced by methods known in the art. In particular, the following method is generally used. First, the codon usage in genes coding for about a dozen proteins which are highly expressed in the intended host cell is determined. Then, the codon usage in genes coding for about a dozen proteins expressed at low levels in the host cell is determined. By reviewing the results, codons that are used often and those that are used rarely are identified. The codons that are used most often in highly-expressed genes are preferred. Those used rarely in highly-expressed genes or used in genes expressed at low levels are preferably not used.

The use of chemically synthesized DNA also allows for the selection of codons with a view to providing unique or nearly unique restriction sites at convenient points in the sequence. The use of these sites provides a convenient means of constructing the synthetic coding sequences and facilitates rapid alteration of the sequences by cassette mutagenesis. In addition, if secondary structures formed by the messenger RNA (mRNA) transcript interfere with transcription or translation, they may be eliminated by altering the codon selections.

Chemical synthesis also allows for the use of optimized expression control sequences with the DNA sequences coding for the mutant proteins. In this manner, optimal expression of the mutant proteins can be obtained. For instance, as noted above, promoters can be chemically synthesized and their location relative to the transcription start optimized. Similarly an optimized ribosome binding site and spacer can be chemically synthesized and used with coding sequences that are to be expressed in prokaryotes.

In prokaryotic mRNA, the site at which the ribosome binds to the messenger includes a sequence of 3-9 purines. The consensus sequence of this stretch is 5'-AGGAGG-3', and it is frequently referred to as the Shine-Dalgarno sequence. The sequence of the ribosome binding site may be modified to alter expression. See Hui and DeBoer, Proc. Natl. Acad. Sci. USA, 84, 4762-66 (1987). Comparative studies of ribosomal binding sites, such as the study of Scherer, et al., Nucleic Acids Res., 8, 3895-3907 (1987), may provide guidance as to suitable base changes.

The ribosome binding site lies 3-12 bases upstream of the start (AUG) codon. The exact distance between the ribosome binding site and the translational start codon, and the base sequence of this "spacer" region, affect the efficiency of translation and may be optimized empirically. To achieve the optimal expression of the mutant proteins of the invention in prokaryotes, a ribosome binding site and spacer that provide for efficient translation in the prokaryotic host cell should be provided. A preferred ribosome binding site and spacer sequence for optimal translation in E. coli are described in Springer and Sligar, Proc. Nat'l Acad. Sci. USA, 84, 8961-65 (1987) and von Bodman et al., Proc. Nat'l Acad. Sci. USA, 83, 9443-47 (1986).

The consensus sequence for the translation start sequence of eukaryotes has been defined by Kozak (Cell, 44, 283-292 (1986)) to be: C(A/G)CCAUGG. Deviations from this sequence, particularly at the -3 position (A or G), have a large effect on translation of a particular mRNA. Virtually all highly expressed mammalian genes use this sequence. Highly expressed yeast mRNAs, on the other hand, differ from this sequence and instead use the sequence (A/Y)A(A/U)AAUGUCU (Cigan and Donahue, Gene, 59, 1-18 (1987)). These sequences may be altered empirically to determine the optimal sequence for use in a particular host cell.

A host cell capable of expressing a mutant protein according to the invention can be prepared by transforming the cell with a vector comprising a DNA sequence that codes for the mutant protein. Alternatively, a DNA molecule coding for a mutant protein may used to transform the host cell. Methods of transforming cells with vectors or DNA are well known in the art.

Any of a large number of available and well-known host cells may be used in the practice of this invention. The selection of a particular host is dependent upon a number of factors recognized by the art. These include, for example, compatibility with the chosen expression vector, toxicity to it of the mutant protein encoded for by the DNA sequence, rate of transformation, expression characteristics, bio-safety and costs. A balance of these factors must be struck with the understanding that not all hosts may be equally effective for the expression of a particular mutant protein.

Within these general guidelines, useful hosts include bacteria (such as E. coli sp.), yeast (such as Saccharomyces sp.) and other fungi, insects, plants, animals (including human), or other hosts known in the art.

The mutant proteins of the invention may be produced by culturing the chosen host cell under conditions which permit expression of the mutant protein. Methods of culture and culture media are well known in the art, but the use of enriched media (rather than minimal media) is preferred since much higher yields are obtained.

### EXAMPLES

Restriction enzymes used in the following examples were obtained from various commercial sources and used according to the manufacturer's instructions or by employing a standard buffer system (Maniatis et al., Molecular Cloning: A Laboratory Manual (1982)).

### EXAMPLE 1: Preparation Of Cys36→Ala, Cys97→Ala Mutant C-Reactive Protein Subunit

This example describes the preparation of a recombinant DNA molecule coding for a mutant human CRP subunit in which the two cysteine residues at positions 36 and 97 (using the numbering system of Woo, et al., J. Biol. Chem., 260, 13384-13388 (1985)) have been replaced with alanine residues and a methionine has been added at the amino terminal end. This example also describes the preparation of vectors containing the recombinant DNA molecule operatively linked to expression control sequences and the expression of the mutant CRP subunits in *Escherichia coli*. Finally, this example describes the properties of the mutant human CRP subunit including, particularly, its ability to bind aggregated immunoglobulin.

### A. Replacement Of The Codons For Cysteine-36 And Cysteine-97 In The CRP Coding Sequence Using A Polymerase Chain Reaction Technique

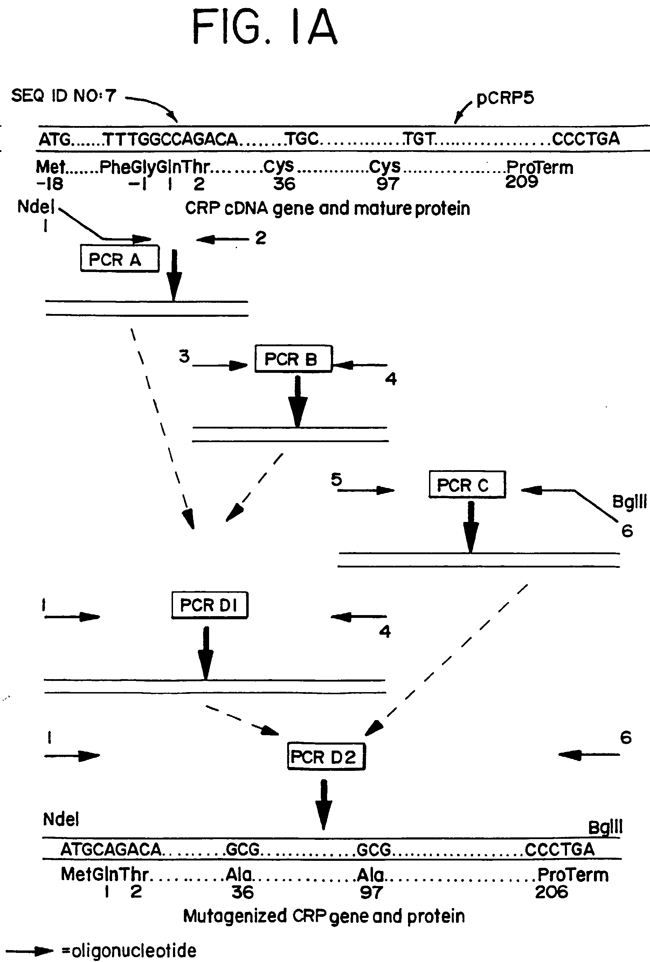

Replacement of the codons for cysteine-36 and cysteine-97 in the coding sequence for mature human CRP subunits with codons for alanine was accomplished using the method of Horton et al., BioTechniques, 8, 528-535 (1990) which is based on the polymerase chain reaction (PCR; Saiki et al., Science, 239, 487-491 (1988)). Since two independent changes were desired, the process was modified to incorporate a total of five PCR reactions as illustrated in Figure 1A. Table 1 shows the sequence of the oligonucleotides used as primers in the PCR reactions.

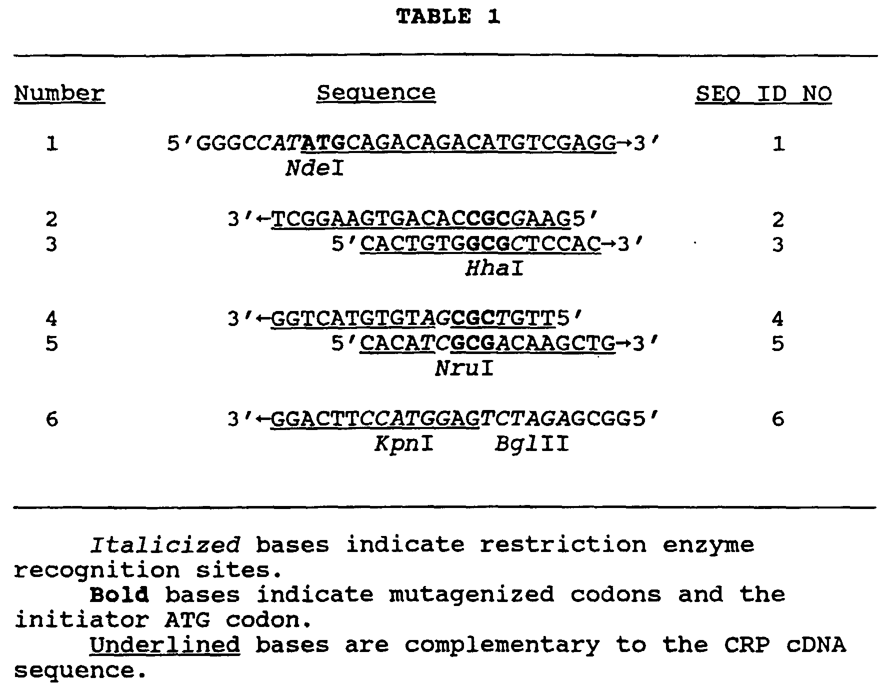

As shown in Figure 1A, the five reactions were: (1) Reaction of cDNA clone coding for preCRP with primers 1 and 2 to produce PCR product A; (2) Reaction of cDNA coding for preCRP with primers 3 and 4 to produce PCR product B; (3) Reaction of cDNA coding for preCRP with primers 5 and 6 to produce PCR product C; (4) Reaction of products A and B in the presence of primers 1 and 4 to produce PCR product D1; and (5) Reaction of products D1 and C in the presence of primers 1 and 6 to produce the final product D2. D2 codes for the mature sequence of human CRP subunit (the presequence has been eliminated), except that there is an additional methionine at the N-terminus and cysteines 36 and 97 have been replaced by alanines.

The DNA coding for human preCRP used as the starting material for these PCR reactions was obtained by digestion of pCRP5 with *EcoRI* to yield linear (noncircular) DNA. Plasmid pCRP5 was obtained from Drs. Bruce Dowton and Harvey Colten of Washington University School of Medicine, St. Louis, MO. pCRP5 was isolated from a human liver cDNA library as described in Tucci et al., J. Immunol., 131, 2416-19 (1983). The nucleotide sequence of the cDNA of pCRP5 and the amino acid sequence of the preCRP coded for by it are given in Woo, et al., J. Biol. Chem., 260, 13384-13388 (1985).

The PCR reactions were carried out using VENT polymerase (New England Biolabs) to minimize unwanted mutations due to misincorporation of bases, and the PCR reactions were done using 20 cycles, each cycle consisting of: 94°C for 1 minute; 37°C, 42°C or 60°C for 1 minute (the annealing temperature depending on the sequence of the primers); and 74°C for 3 minutes. Following the amplification steps, the reactants were further incubated at 74°C for 5 minutes to complete the synthesis of double-stranded DNA. Each of the products was purified by agarose gel electrophoresis as described in Horton et al., supra. For the PCR reactions where the template consisted of two overlapping sequences (PCR D1 and PCR D2 in Figure 1A), the reactants were incubated without primers for 4 cycles to allow the formation of full-length template before normal amplification was carried out.

PCR products were digested with restriction endonucleases *HhaI* and *NruI* (see Table 1) to confirm that the products incorporated the desired mutations.

### B. Construction Of A Plasmid For The Overexpression of The Mutant CRP Subunit

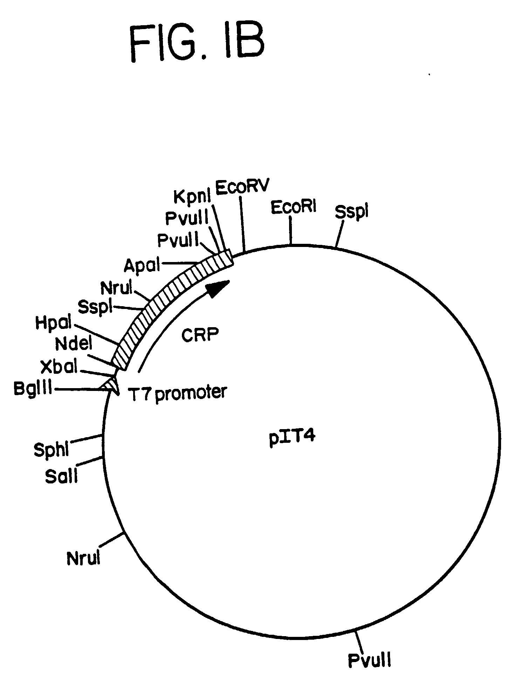

The final PCR product D2 was concentrated by filtration through a Centricon 30 apparatus (Amicon, Beverly, MA), and then treated with T4 polynucleotide kinase (Pharmacia, Piscataway, NJ) and T4 DNA ligase (New England Biolabs, Inc.) as described in Denney et al., Amplifications, 4, 25-26 (1990). The resultant material was digested with *NdeI* and *BglII* to release the mutant CRP coding sequence, and the released coding sequence was ligated to the expression vector pETV which had been digested with *NdeI* and *BamHI* and treated with calf intestinal alkaline phosphatase (Promega, Madison, WI). The ligation mixture was used to transform *E. coli* DH5α (Gibco BRL Life Technologies, Inc.), and transformants were screened by minipreps performed as described in Birnboim et al., Nucleic Acids Res., 7, 1513-1523 (1979) to identify the correct plasmid pIT4. A restriction map of pIT4 is provided in Figure 1B. As shown in Figure 1B, the mutant CRP coding sequence is under the control of the T7 promoter.

Plasmid pETV is a derivative of pET3a. The preparation of pET3a is described Rosenberg et al., Gene, 56, 125-135 (1987). Plasmid pET3a was obtained from Dr. W. Studier, Brookhaven National Laboratory, Upton NY. Plasmid pET3a has two *NheI* restriction enzyme sites. One is located at the T7 gene *10* translation start site in which it was desired to insert the mutant CRP coding sequence. The second *NheI* site is located within a 190-bp fragment bounded by *Eco*RV sites. This second *NheI* site was eliminated by digestion with *Eco*RV and recircularizing the plasmid to yield pETV. The predicted sequence of the junction between the expression system of pETV and the coding region for mutant CRP subunit in pIT4 was confined by DNA sequencing as follows. Plasmid pIT4 was digested with *Xba*I and *Kpn*I, and the relevant fragment subcloned into M13mp18RF (Yanisch-Perron et al., Gene, 33, 103-119 (1985)). Single-stranded DNA was obtained from cultures carrying M13mp18RF and sequenced as described by Sanger et al., J. Mol. Biol., 94, 441-558 (1975) using an Applied Biosystems Model 370A Automated DNA Sequencer.

### C. Expression And Purification Of The Mutant CRP Subunit

Plasmid pIT4 was used to transform *E. coli* BL21(DE3) (preparation described in Studier et al., J. Mol. Biol., 189, 113-130 (1986)) which carries the phage T7 RNA polymerase gene under expression control of the *lac*UV5 operator and promoter. Competent *E. coli* BL21(DE3) was obtained from Novogen, Madison, WI. Transformants were selected on LB medium (Miller, Experiments In Molecular Genetics (1972)) containing 50 µg/ml ampicillin.

Transformed cells were grown in M9ZB medium (Studier et al., J. Mol. Biol., 189, 113-130 (1986)) containing 100 µg/ml ampicillin at 37°C for small-scale experiments. Ten-liter cultures were grown in a New Brunswick Microgen SF-116 fermentor in 2YT medium (Miller, Experiments In Molecular Genetics (1972)) plus 0.4% (w/v) glucose and 100µg/ml ampicillin at 37°C with aeration using compressed air at a rate of 10 liters per minute.

Synthesis of the T7 RNA polymerase, and consequently of the mutant CRP subunit, was induced with 1 mM (final concentration) isopropyl-beta-D-thiogalactoside (IPTG; Boehringer Mannheim) when the cell density reached OD₆₀₀ = 4. The cells were harvested 3 hours after induction by rapidly mixing the culture with an equal volume of ice, concentrating the cells by filtration using a Millipore Pellicon apparatus equipped with a Durapore 0.5 µM membrane, and centrifuging the cells in a Beckman JA10 rotor at 10,000 rpm for 20 min.

The harvested cells were suspended in 20mM Tris-HCl, pH 7.5, containing 5mM EDTA (40 g in 500 ml) and disrupted by three passages through a Manton-Gaulin homogenizer at 10,000 psi. The extract was centrifuged in a Beckman JA10 rotor at 8,000 rpm for 20 min. at 2°C. The pellet containing the insoluble mutant CRP subunits was washed twice with 20 ml of the same buffer containing 0.5% (v/v) Triton X-100 (Bio-Rad), followed by centrifugation in a JA18 rotor at 9,000 rpm for 20 min. to remove the soluble material.

About 5-6 mg of the final pellet were resuspended in 50-70 ml. of 8M ultra-pure urea (Boehringer Mannheim, Indianapolis, IN) in 10 mM Tris-HCl, pH 7.5-8.0, and incubated at 4°C overnight with mixing to solubilize the mutant CRP subunits (this material is referred to hereinafter as "mutant rCRP inclusion body preparation"). Next, this material was diluted with 10mM Tris-HCl, pH 7.5-8.0, to a final concentration of 6M urea and then loaded onto a column containing 40 cc of Q-Sepharose Fast Flow^{R} anion exchange resin (Pharmacia) at 6 ml per minute. Bound materials were eluted with a linear NaCl gradient using 10mM Tris-HCl, pH 7.5-8.0, containing 6M urea and 1 M NaCl. Absorbance at 280 nm was measured using a BioPilot^{R} automated chromatography system (Pharmacia), and an elution profile was obtained.

For comparison, native CRP, mCRP and the primary translation product of a CRP cDNA clone (hereinafter "wild-type rCRP") were also chromatographed on Q-Sepharose Fast Flow^{R}. Native CRP, mCRP and wild-type rCRP were prepared and chromatographed as follows.

Native CRP was isolated from pleural or ascites fluid by calcium-dependent affinity chromatography using phosphorylcholine-substituted BioGel^{R} A 0.5m (an agarose-based resin obtained from BioRad Laboratories) as described by Volanakis, et al. [J. Immunol., 113, 9-17 (1978)] and modified by Potempa, et al. [Mol. Immunol., 24, 531-41 (1987)]. Briefly, the pleural or ascites fluid was passed over the phosphorylcholine-substituted column, and the CRP was allowed to bind. Then, the column was exhaustively washed with 75 mM Tris-HCl-buffered saline (pH 7.2) containing 2 mM CaCl₂ until the absorbance at 280 nm was less than 0.02. The CRP was eluted with 75 mM Tris, 7.5 mM citrate-buffered saline (pH 7.2). This high concentration of Tris significantly reduces non-specifically adsorbed proteins which often contaminate affinity-purified CRP preparations. CRP-containing fractions were pooled, diluted three-to-five fold with deionized water, adsorbed to Q-Sepharose Fast Flow^{R} ion exchange resin, and then eluted with a linear salt gradient of from 0-1 M NaCl in 10mM Tris-HCl, pH 7.4. CRP-containing fractions were pooled and recalcified to 2-5mM CaCl₂ (by adding a suitable amount of a 1M solution) and applied to unsubstituted Biogel^{R} A 0.5m column to remove residual serum amyloid P component (SAP). Then, the CRP was concentrated to 1 mg/ml using ultrafiltration (Amicon; PM30 membrane) under 10-20 psi nitrogen. A CRP extinction coefficient (mg/ml) of 1.98 was used to determine concentration. Next, the concentrated CRP was exhaustively dialyzed against 10 mM Tris-HCl-buffered saline, pH 7.2, containing 2mM CaCl₂. This preparation produced a single Mr 23,000 band on SDS-PAGE electrophoresis and was more than 99% free of SAP, IgG and all other proteins tested for antigenically.

For the Q-Sepharose Fast Flow^{R} column, 5 ml of the final concentrated CRP solution containing 5 mg of purified native CRP was diluted in about 30 ml of 10 mM Tris-HCl, pH 7.4, and the resulting solution loaded on the Q-Sepharose Fast Flow column at 6 ml per minute. Bound material was eluted with a linear NaCl gradient using 10mM Tris-HCl, pH 7.4, containing 1 M NaCl. A₂₈₀ was measured using the BioPilot^{R} automated chromatography system.

To make mCRP, purified native CRP, prepared as described above, at 1 mg/ml was incubated in 8M ultrapure urea in the presence of 10mM EDTA for one hour at 37°C. For the Q-Sepharose Fast Flow^{R} column, 6 ml of this material containing 6 mg of the mCRP was diluted in about 30 ml of 10 mM Tris-HCl, pH 7.5-8.0, containing 6M ultra pure urea, and the resulting solution was loaded on the Q-Sepharose Fast Flow^{R} column at 6 ml per minute. Bound material was eluted with a linear NaCl gradient using 10mM Tris-HCl, pH 7.5-8.0, containing 6M urea and 1 M NaCl. A₂₈₀ was measured using the BioPilot^{R} automated chromatography system.

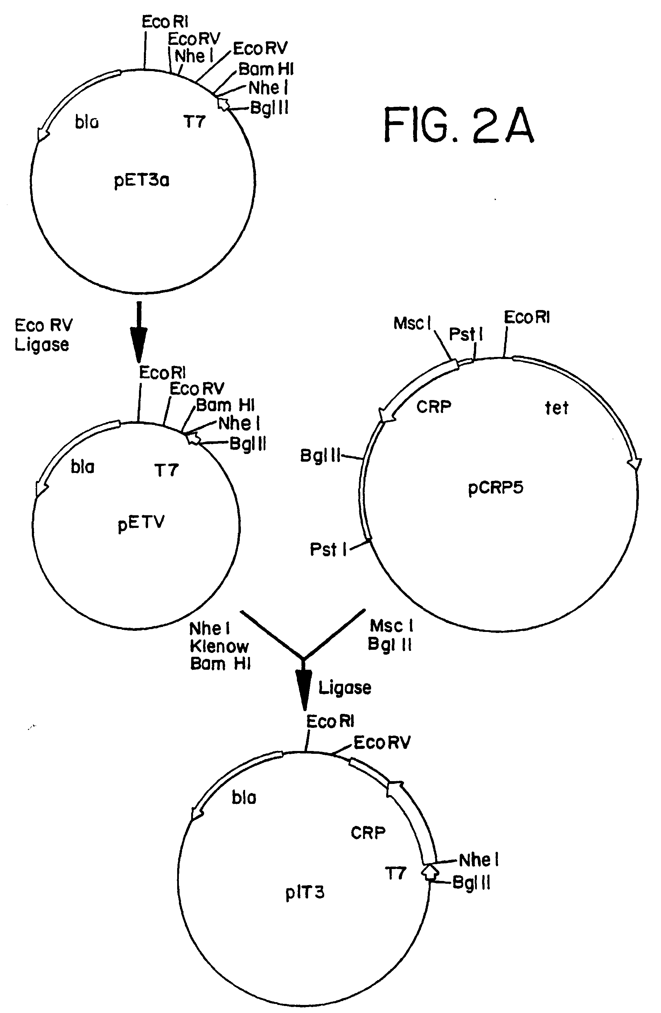

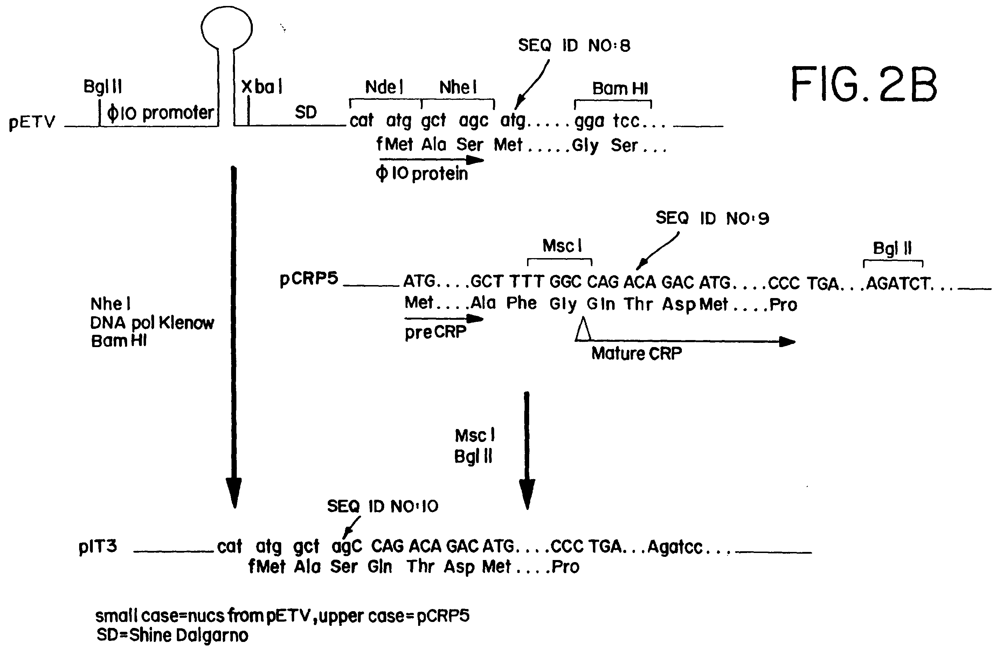

The wild-type rCRP was prepared by expression of pIT3 in *E. coli* BL21(DE3). Plasmid pIT3 was prepared by cleaving pCRP5 with *Msc*I and *Bgl*II (see Figure 2A). Next, pETV was cleaved with *Nhe*I and *Bam*HI, and the digested pETV and pCRP5 were mixed and ligated (see Figure 2A). This ligation mixture was used to transform *E. coli* strain DH5α and colonies carrying the desired expression plasmid pIT3 were identified, all as described above for pIT4. Plasmid pIT3 codes for a CRP subunit having the sequence of the unmutated human CRP subunit, except that it has the peptide Met Ala Ser at the N-terminus (see Figure 2B). *E. coli* BL21(DE3) were transformed with pIT3 and cultured as described above for pIT4. The cultures were induced, the cells were harvested, and a pellet containing the wild-type rCRP was obtained, all as described above. About 7.4 mg of the pellet were resuspended in 50-70 ml. of 8M urea in 10 mM Tris-HCl, pH 7.5-8.0, and incubated at 4°C overnight with mixing (this material is referred to hereinafter as "wild-type rCRP inclusion body preparation"). The solubilized inclusion body preparation was passed through filter paper to remove non-solubilized debris. Next, this material was diluted with 10mM Tris-HCl, pH 7.5-8.0, to a final concentration of 6M ultra pure urea and then loaded onto the Q-Sepharose Fast Flow^{R} column at 6 ml per minute. Bound materials were eluted with a linear NaCl gradient using 10mM Tris-HCl, pH 8.0, containing 6M urea and 1 M NaCl. A₂₈₀ was measured using the BioPilot^{R} system.

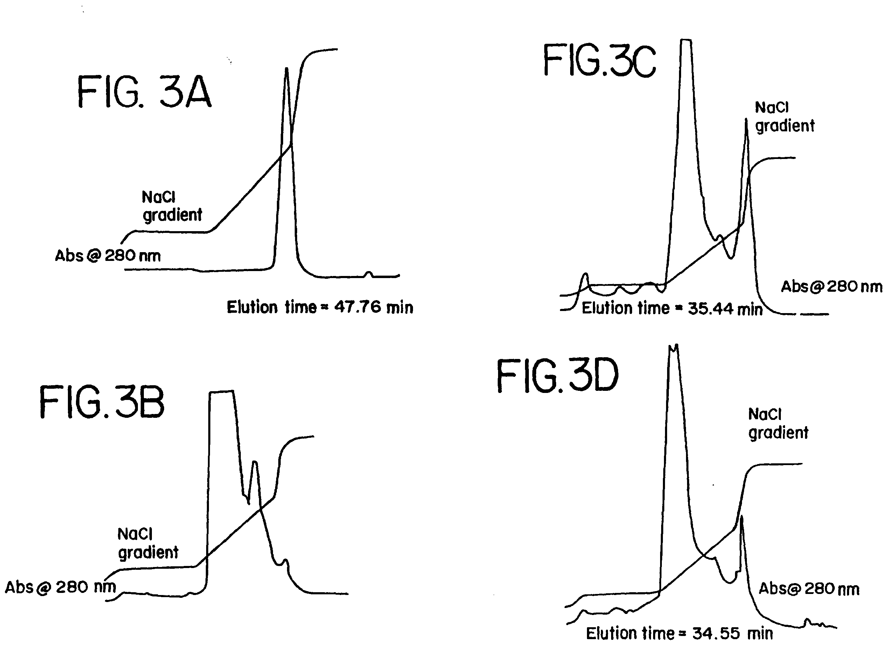

The elution profiles generated by the BioPilot^{R} system for native CRP, mCRP, wild-type rCRP and the mutant CRP subunits are shown in Figures 3A-D. The BioPilot^{R} system was programmed so that the salt gradient used in each case could be directly compared.

As shown in Figure 3A, native CRP gave only one significant elution peak. It had an elution time of 47.76 minutes. This peak was dialyzed against 25mM Tris-HCl, 0.15M NaCl, 2 mM CaCl₂, pH 7.4, and then concentrated to about 1 mg/ml using Amicon filtration for testing as described in the next section. The concentrated peak material is referred to hereinafter as "native-CRP_{Q}".

Figure 3B shows the elution profile for mCRP. The predominant peak has an elution time of 36.18 minutes. This peak was dialyzed against low ionic strength buffer (25 mM Tris-HCl, 0.015 M NaCl, pH 7.4) and then concentrated using Amicon filtration and was tested as described in the next section. The concentrated peak material is referred to hereinafter as "mCRP_{Q}". The testing described in the next section verified that the material in the peak was mCRP. Hence, mCRP elutes from Q-Sepharose^{R} much earlier (approximately 11.5 minutes earlier) than native CRP.

Figure 3C shows the elution profile for wild-type rCRP. The predominant peak eluted at 34.55 minutes, an elution time almost identical to that of mCRP and very distinct from the elution time of native CRP. The protein in the predominant peak was dialyzed against low ionic strength buffer and then concentrated using Amicon filtration for further testing as described in the next section. The concentrated peak material is referred to hereinafter as "wild-type rCRP_{Q}".

Finally, Figure 3D shows the elution profile for the mutant CRP subunits. The main peak eluted at 34.55 minutes, an elution time essentially identical to that of wild-type rCRP and mCRP, and distinct from that of native CRP. The main peak was dialyzed against low ionic strength buffer and then concentrated using Amicon filtration for further testing as described in the next section. The concentrated peak material is referred to hereinafter as "mutant rCRP_{Q}".

Proteins may also be eluted from the Q-Sepharose Fast Flow^{R} column with an NaCl step gradient (from 0.1 to 1.0 M NaCl). When the recombinant mutant CRP protein was run on the column and eluted with a step gradient, the A₂₈₀ readings showed that most of the protein was eluted from the column with 0.1 - 0.2 M NaCl. When native CRP was run on the column and eluted with a step gradient, the A₂₈₀ readings showed that most of the protein eluted with 0.4 M NaCl.

### D. Characterization Of The Fractions Eluted From The O-Sepharose Fast Flow^{R} Column

The peaks from the Q-Sepharose Fast Flow column were analyzed by dot blot, Western blot, SDS-PAGE and ELISA. Wild-type rCRP and mutant rCRP inclusion body preparations were sometimes also tested. Native CRP and mCRP were sometimes used as controls. The preparation of all of these materials is described in the previous section.

### 1. Dot Blot Assays

Following a modification of the procedure of Zhang, J. Immunol., 138, 575 (1987), nitrocellulose membranes (Schleicher & Schuell, Keene, NH) were presoaked in TBS (25 mM Tris-HCl, 0.15M NaCl, pH 7.4) for 30 min., and excess buffer was removed with filter paper. The membranes were then fitted into a Bio-Dot™ microfiltration apparatus (Bio-Rad). Aliquots (50 µl) of the various test proteins at 5 µg/ml were dotted onto the membrane, incubated overnight at 4°C, and then vacuum-filtered to remove all of the liquid from the wells. Blocking solution (100 µl of 1% BSA in TBS) was added to the wells and incubated for 30 min. at room temperature (RT), and vacuum-filtered through the membrane. The wells were washed three times with TBS containing 1% BSA and 0.05% Tween 20 (TBS washing buffer). Mouse monoclonal antibody (mAb) 3H12 and antiserum LP3-HRP were added and incubated for 30 min. at RT followed by washing. Monoclonal antibody 3H12 was used unlabeled, and the blots were developed by adding rabbit anti-mouse IgG F(ab')₂ labeled with horseradish peroxidase (Southern Biotechnology Associates, Birmingham, AL), incubating for 30 min. at RT, adding peroxidase substrate 4-chloro-2-naphthol (Bio-Rad) in 10 mM Tris-HCl, 0.15 M NaCl, containing methanol and H₂O₂ prepared as directed (Bio-Rad), and incubating for 30 min. at RT to allow for color development. LP3-HRP was labeled with horseradish peroxidase, and the blots were developed by adding 4-chloro-2-naphthol followed by an incubation for 30 min. at RT for color development.

Monoclonal antibody 3H12 is an IgG antibody specific for an antigenic determinant found on mCRP but not on native CRP. Its preparation and properties are described in published PCT application WO 91/00872 and in Ying et al., J. Immunol., 143, 221-228 (1989). Antiserum LP3 is an antiserum prepared by immunizing a goat with mCRP in complete Freund's adjuvant and then affinity-purifying the harvested antiserum by passing it over a column of cyanogen bromide-activated BioGel^{R} substituted with mCRP. The resulting affinity-purified anti-neoCRP antiserum LP3 was monospecific for the neo-CRP antigenicity expressed by mCRP but not by native CRP. LP3 was labelled with horseradish peroxidase as described in Potempa et al., Molec. Immunol., 24, 531-541 (1987).

The dot blots showed that mCRP, mCRP_{Q}, wild-type rCRP_{Q} and mutant rCRP_{Q} reacted with monoclonal antibody 3H12 and monospecific anti-neoCRP LP3-HRP, indicating that all of these materials express antigenic determinants found on mCRP but not on native CRP. Native CRP and native-CRP_{Q} did not react with antibodies 3H12 and LP3-HRP as expected.

### 2. Western Blot

The peaks were also analyzed by Western blot. To perform the Western blot, 5-10 µl of the peak concentrates were electrophoresed on 12% SDS-PAGE gels under reducing and non-reducing conditions. After electrophoresis, protein was transferred to a nitrocellulose membrane using the JKA Biotech (Denmark) Semidry Electroblotter. The remainder of the procedure was the same as described above for the dot blot, except that three mouse mAbs (3H12, 2C10 and 8C10) were used. The color was developed as described in the previous section for 3H12.

Monoclonal antibody 3H12 is described above. Monoclonal antibody 8C10 reacts with a determinant found only on mCRP, whereas 2C10 reacts with a determinant found on both native CRP and mCRP. The preparation and properties of 2C10 and 8C10 are described in published PCT application WO 91/00872 and in Ying et al., J. Immunol., 143, 221-228 (1989).

The Western blot results showed that mCRP, mCRP_{Q}, wild-type rCRP_{Q} and mutant rCRP_{Q} reacted with all three mAbs, indicating that all of these materials express antigenic determinants found on mCRP. Native CRP and native-CRP_{Q} reacted with mAb 2C10, but not with mAb 3H12 and 8C10, confirming that these materials were native CRP and the antibodies were reacting as expected.

The Western blot results also showed that the predominant material present in mutant rCRP_{Q} (the major peak obtained when the mutant CRP subunits were chromatographed on Q-Sepharose Fast Flow^{R}) was free, monomeric subunits. Some multimers and fragments reactive with the antibodies specific for mCRP determinants were present, but the number of such undesired bands was much fewer than was observed with wild-type rCRP_{Q}. This indicates that by replacing the cysteine residues in a CRP subunit, the resultant recombinant product can be more efficiently processed to give a purer, more well-defined product.

### 3. SDS-PAGE

The peak concentrates were run on PhastGel^{R} SDS-PAGE gels (Pharmacia). A gradient of 8-25% gradient acrylamide was used. After the electrophoresis was complete, the gels were stained with Coomassie blue.

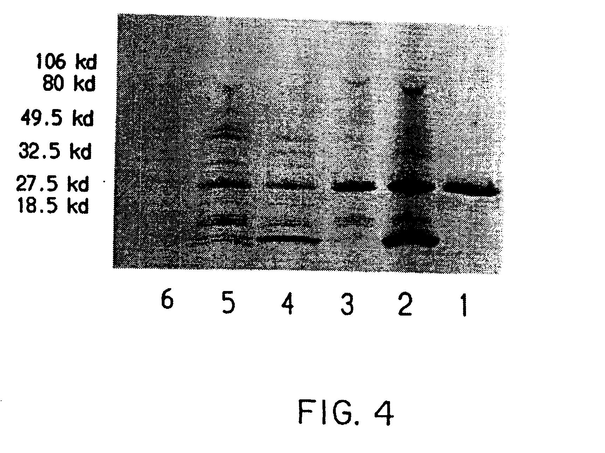

The results are shown in Figure 4. In Figure 4, lane 1 contains mCRP. A single band at approximately 27,000 molecular weight was obtained.

Lane 2 contains the mutant rCRP inclusion body preparation. Note that there are two predominant bands, including one at approximately the same position as the single mCRP band (Mr of about 27,000). This band was verified by Western blot analysis to be antigenically reactive with antibodies specific for mCRP determinants.

Lane 3 contains mutant rCRP_{Q} (the mutant rCRP inclusion body preparation which had been chromatographed on Q-Sepharose Fast Flow^{R}). Note that only one major band was obtained having a molecular weight of approximately 27,000, the molecular weight of the desired free mutant CRP subunits. As can be seen, there are many fewer bands as compared to the mutant rCRP inclusion body preparation (lane 2), and the amounts of remaining contaminants, especially the one predominant contaminant having Mr less than 18,000, have been substantially reduced. Thus, the purity of the mutant CRP subunit was greatly improved by the single-step Q-Sepharose chromatography procedure.

Lane 4 contains wild-type rCRP inclusion body preparation. As can be seen, multiple protein bands are present. The band approximately 80% down the lane is the free, intact wild-type rCRP subunit (Mr of about 27,000). Note that it is not the predominant band, with the band at the bottom of the lane being of greater intensity.

Lane 5 contains wild-type rCRP_{Q} (the wild-type rCRP inclusion body preparation which had been chromatographed on Q-Sepharose Fast Flow^{R}). As can be seen, multiple bands are still present as compared to the unchromatographed inclusion body preparation (lane 4). Indeed, some new bands have appeared (at approximate molecular weights of 45,000 and 14,000), the identity of which is unknown, and the intensity of some bands has increased. Thus, no improvement in purification of wild-type rCRP was achieved using the Q-Sepharoae Fast Flow^{R} column. The band about 80% down the lane is the free subunit. This band and many of the other bands were reactive with the antibodies specific for mCRP determinants by Western blot analysis. This indicates that most of the protein recovered after chromatography of wild-type rCRP preparations on the Q-Sepharose Fast Flow^{R} column is wild-type rCRP, but that there are problems of multimerization and fragmentation.

Lane 6 contains Prestained BioGel^{R} molecular weight standards (BioRad). From top to bottom, these bands are of approximate molecular weight 106,000, 80,000, 49,500, 32,500, 27,500 and 18,500.

### 4. ELISA

Finally, an ELISA was performed to detect binding of antibodies specific for native CRP and mCRP to the materials in the peaks eluted from the Q-Sepharose Fast Flow^{R} columns. A direct binding ELISA was used for mCRP and recombinant CRP preparations. A ligand capture ELISA was used for native CRP preparations.

In the direct ELISA, 100 µl of each test protein (5 µg/ml) in 50mM sodium bicarbonate buffer (pH 9.5) were placed in the wells of Nunc polystyrene plates (Scientific Supply, Shiller Park, IL) and incubated for 2 hr at 37°C or 4°C overnight. The wells were blocked with TBS (25mM Tris-HCl, 0.15M NaCl, pH 7.4) containing 1% bovine serum albumin (BSA) (TBS-A) for 60-120 min. at 37°C. The wells were washed with TBS containing 0.05% Tween 20 (TBS-wash buffer). Antibodies were serially diluted with TBS-A, and 100 µl aliquots were added to the wells and incubated for 60 min. at 37°C, followed by washing. Peroxidase-conjugated rabbit anti-mouse IgG (Southern Biotech) in TBS-A was added to the wells for 60 mm. at 37°C. After washing, 100 µl ABTS substrate (2-2'azino-bis(3-ethylbenzylthiazoline-6-sulfonic acid, Sigma Chemical Co.) were added per well and incubated for about 5-15 min. at RT. Plates were read at an absorbance of 414 nm on a Titertek^{R} multiskan plate reader (Flow Laboratories, Helsinki, Finland).

For the ligand capture ELISA, plates were incubated with 100 µl/well of PC-KLH (5 µg/ml) in bicarbonate buffer for 2 hr. at 37°C or overnight at 4°C. Wells were blocked with TBS-A containing 2 mM CaCl₂ as described above. After blocking, 100 µl/well of native CRP (5 µg/ml) in TBS-A containing 2mM CaCl₂ were added and incubated for 60 min. at 37°C. After washing, the rest of the assay was performed as described above, except that 2mM CaCl₂ was included in all buffers. PC-KLH is phosphorylcholine (PC) substituted Keyhole Limpet hemocyanin (KLH). It was prepared by incubating KLH (Sigma Chemical Co.) with paranitrophenyl phophorylcholine (Sigma Chemical Co.) diazotized as described by Chesebro and Metzger, Biochemistry, 11, 766 (1972). The final derivatization resulted in 28-52 moles PC per Mr of 1 x 10⁻⁵ KLH.

The results of these ELISAs are shown in Figures 5A-D. Figure 5A shows the reactivity of mCRP, wild-type rCRP inclusion body preparation and native CRP with mAb 3H12. As expected mAb 3H12 reacted with mCRP but not with native CRP. Also, wild-type rCRP inclusion body preparation reacted like mCRP and unlike native CRP, indicating that the mCRP epitope recognized by mAb 3H12 (the carboxy-terminal octapeptide of the CRP subunit) is expressed on wild-type rCRP.

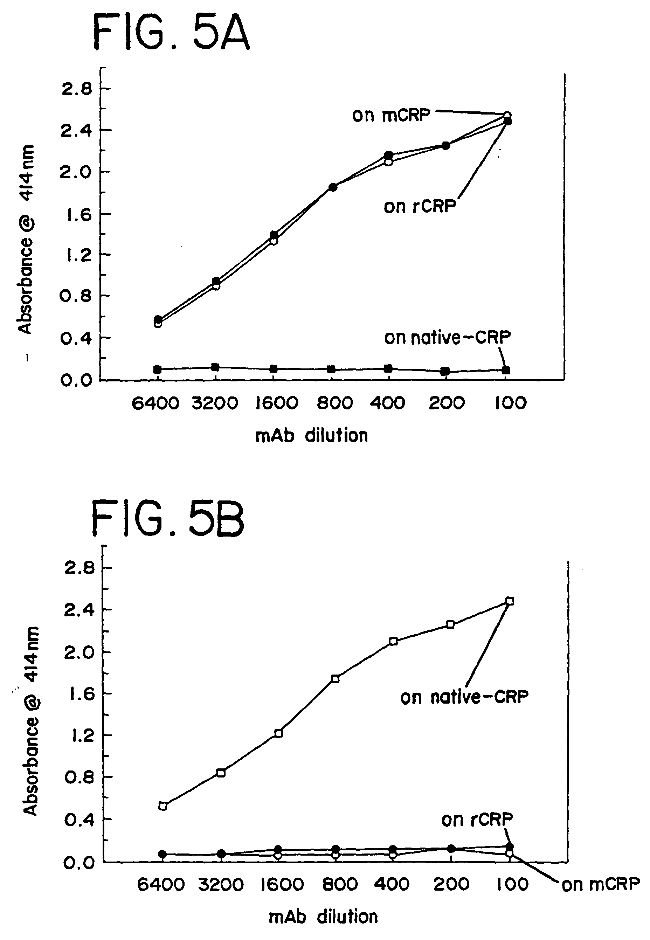

Figure 5B shows the reactivity of mCRP, wild-type rCRP inclusion body preparation and native CRP with mAb 1D6. Monoclonal antibody 1D6 is specific for an antigenic determinant found only on native CRP. Its preparation and properties are described in published PCT application WO 91/00872 and in Ying et al., J. Immunol., 143, 221-228 (1989). As expected mAb 1D6 reacted with native CRP but not with mCRP. As shown, wild-type rCRP inclusion body preparation, like mCRP, did not react with mAt 1D6, indicating that the native CRP epitope recognized by mAb 1D6 is not expressed on wild-type rCRP.

Figure 5C shows the reactivity of mCRP, wild-type rCRP_{Q} and mutant rCRP_{Q} with mAb 8C10. Monoclonal antibody 8C10 reacts with an epitope found only on mCRP, but reacts with a different epitope on mCRP than does mAb 3H12. For this ELISA, each test protein was adjusted so that 1000 ng of protein was immobilized on the first well of the polystyrene ELISA plate. The test proteins were then serially diluted so that less and less protein was immobilized per well. The amount of protein that was immobilized per well is indicated on the abscissa of the graph in Figure 5C. As shown, both wild-type rCRP_{Q} and mutant rCRP_{Q} reacted with mAb 8C10 in a manner similar to mCRP. This suggests that the recombinant proteins express an 8C10-specific epitope similar to the one found on mCRP.

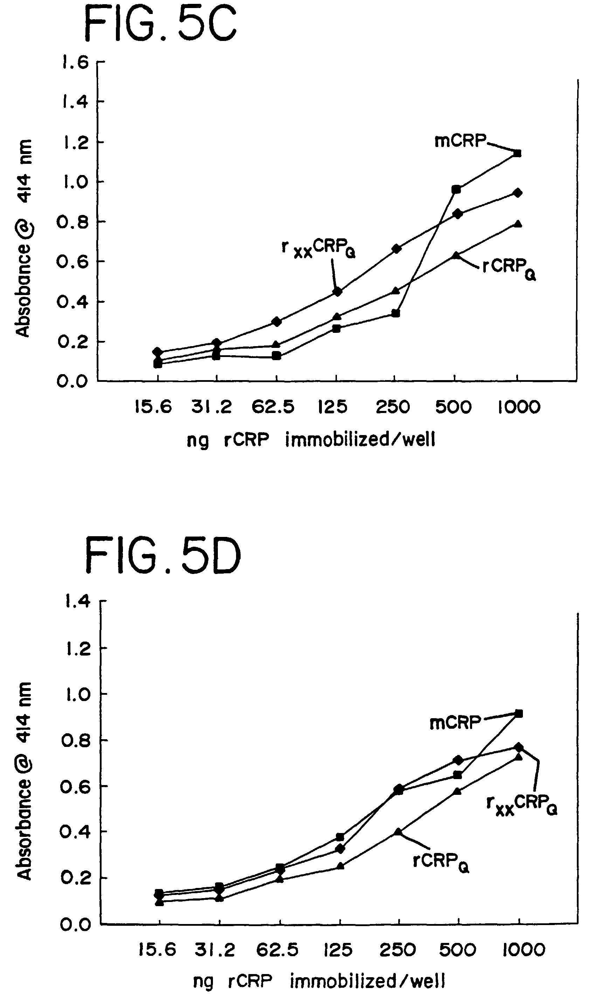

Figure 5D shows the reactivity of mCRP, wild-type rCRP_{Q} and mutant rCRP_{Q} with affinity-purified antiserum LP3-HRP specific for neo-CRP antigenicity. For this ELISA, each test protein was adjusted so that 1000 ng of protein was immobilized on the first well of the polystyrene ELISA plate. The test proteins were then serially diluted so that less and less protein was immobilized per well. The amount of protein that was immobilized per well is indicated on the abscissa of the graph in Figure 5D. As shown, both wild-type rCRP_{Q} and mutant rCRP_{Q} reacted with goat anti-neoCRP LP3-HRP in a manner similar to mCRP. This suggests that the recombinant proteins are antigenicly very similar to mCRP.

### E. Evaluation Of The Biological Activity Of The Mutant CRP Subunits

Finally, an ELISA was performed to detect binding to aggregated IgG of the material in the peaks from the Q-Sepharose Fast Flow^{R} columns. Binding to aggregated immunoglobulins and immune complexes is an important activity of mCRP which permits it to be used to removed aggregated immunoglobulins and immune complexes from fluids and to quantitate immune complexes. This ELISA was performed as follows.

One hundred microliters of each test protein (10 µg/ml) in 10mM sodium bicarbonate buffer (pH 9.5) were placed in the wells of polystyrene microtiter plates and incubated for 2 hr. at 37° or overnight at 4°C. The wells were blocked with TBS-A for 60-120 min. at 37°C. The wells were washed with TBS wash buffer. Then, 100 µl of aggregated human IgG or monomeric IgG at varying concentrations in TBS-A were added and incubated for 60 min. at 37°C. Human immune globulin (U.S.P.-Gammar^{R}, Armour Pharmaceutical Co.) was diluted to 20 mg/ml in buffer at pH 9.0 and was aggregated by heating to 63°C for about 30 minutes. Non-aggregated monomeric IgG was separated from the aggregated IgG by molecular sieve chromatography using BioGel^{R} A 1.5m in 25 mM Tris-HCl, 0.3M NaCl, pH 7.4 at 4°C. After monomeric or aggregated IgG was incubated on test-protein-coated wells, the wells were washed with TBS wash buffer, and 100 µl of peroxidase-conjugated goat anti-human IgG F(ab')₂ (Cappel, Durham, NC) in TBS-A were added to the wells and incubated for 60 min. at 37°C. After washing, 100 µl ABTS substrate were added per well and incubated for 5-15 min. at RT. Plates were read at an absorbance of 414 nm on a Titertek^{R} multiskan plate reader.

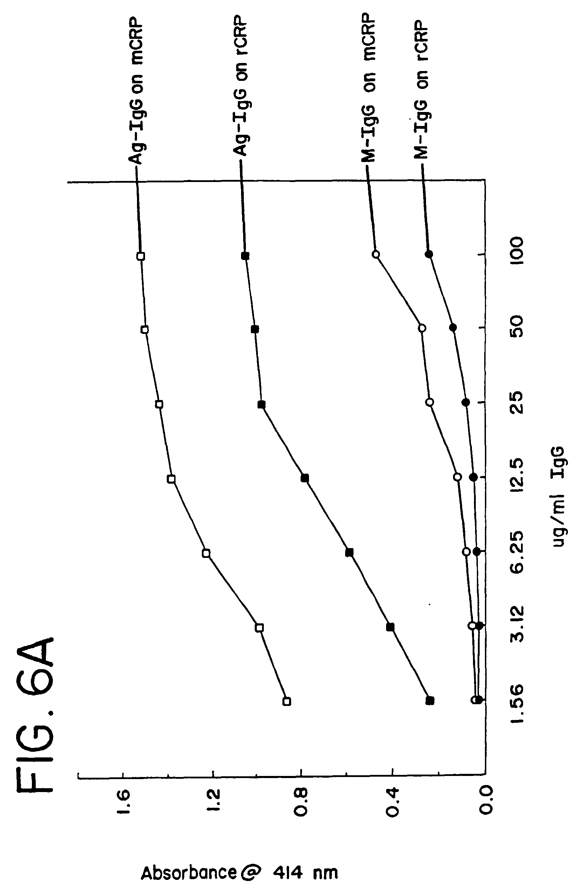

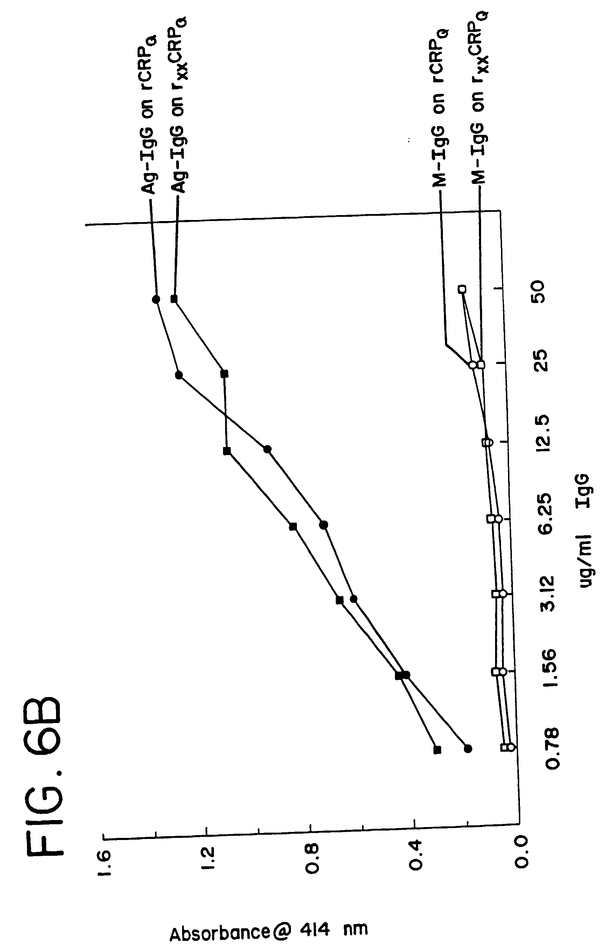

The results are shown in Figures 6A-B. As shown in Figure 6A, wild-type rCRP inclusion body preparation bound aggregated IgG, but not monomeric IgG. Its reactivity was similar to that of mCRP. Figure 6B shows that both Q-Sepharose-fractionated wild-type rCRP_{Q} and mutant rCRP_{Q} bound aggregated IgG, but not monomeric IgG. Hence, both wild-type and mutant recombinant CRPs reacted functionally like mCRP in their capacity to selectively bind aggregated immunoglobulin. Since aggregated immunoglobulin is a standard model for immune complexes, these results also indicate that the mutant proteins of the invention will bind immune complexes and will bind them selectively in the presence of monomeric immunoglobulin.

## Claims

1. A mutant protein which has the same amino acid sequence as the amino acid sequence of an unmutated C-reactive protein (CRP) subunit or an unmutated preCRP, except that
(i) at least one amino acid of the unmutated CRP subunit or unmutated preCRP has been deleted, or
(ii) at least one amino acid of the unmutated CRP subunit or unmutated preCRP has been replaced by another amino acid, or
(iii) at least one amino acid has been added to the unmutated CRP subunit or unmutated preCRP, or
(iv) a combination of such changes has been made,
the amino acid(s) added, the amino acid(s) deleted and the replacement amino acid(s) being chosen so that the mutant protein is less likely to form covalently cross-linked aggregates than the unmutated CRP subunit or unmutated preCRP,
the mutant protein also reacting with an antibody specific for neo-CRP antigenicity and exhibiting at least one of the biological activities of modified-CRP.

2. The mutant protein of claim 1 wherein at least one of the cysteines of the unmutated CRP subunit or unmutated preCRP has been deleted or replaced by another amino acid.

3. The mutant protein of claim 1 wherein the amino acid(s) added, the amino acid(s) deleted and the replacement amino acid(s) are also chosen so that the mutant protein is more soluble in aqueous media than the unmutated CRP subunit or unmutated preCRP.

4. The mutant protein of claim 1 wherein at least one of the lysines of the unmutated preCRP or unmutated CRP subunit has been deleted or replaced by another amino acid.

5. A mutant protein which has the same amino acid sequence as the amino acid sequence of an unmutated C-reactive protein (CRP) subunit, except that
(i) at least one amino acid of the unmutated CRP subunit has been deleted, or
(ii) at least one amino acid of the unmutated CRP subunit has been replaced by another amino acid or
(iii) at least one amino acid has been added to the unmutated CRP subunit, or
(iv) a combination of such changes has been made,
the amino acid(s) added, the amino acid(s) deleted and the replacement amino acid(s) being chosen so that the mutant protein is less likely to form covalently cross-linked aggregates than the unmutated CRP subunit,
the mutant protein also reacting with an antibody specific for neo-CRP antigenicity and exhibiting at least one of the biological activities of modified-CRP.

6. The mutant protein of claim 5 wherein at least one of the cysteines of the unmutated CRP subunit has been deleted or replaced by another amino acid.

7. The mutant protein of claim 6 wherein at least one of the cysteines of the unmutated CRP subunit has been replaced by an amino acid selected from the group consisting of alanine, glycine, valine, leucine, isoleucine, serine, methionine, and threonine.

8. The mutant protein of claim 7 wherein at least one of the cysteines of the unmutated CRP subunit has been replaced by an alanine.

9. The mutant protein of claim 6 wherein all of the cysteines of the unmutated CRP subunit have been deleted or replaced by another amino acid.

10. The mutant protein of claim 9 wherein all of the cysteines of the unmutated CRP subunit have been replaced by an amino acid selected from the group consisting of alanine, glycine, valine, leucine, isoleucine, serine, methionine, and threonine.

11. The mutant protein of claim 10 wherein all of the cysteines of the uninutated CRP subunit have been replaced by alanines.

12. The mutant protein of claim 5 wherein at least one of the lysines of the unmutated CRP subunit has been deleted or replaced by another amino acid.

13. A DNA molecule coding for the mutant protein of any one of claims 1-12.

14. A vector for expression of the mutant protein of any one of claims 1-12 comprising DNA coding for the mutant protein operatively linked to expression control sequences.

15. A host cell which has been transformed so that it contains DNA coding for the mutant protein of any one of claims 1-12, the DNA coding for the mutant protein being operatively linked to expression control sequences.

16. A method of producing the mutant protein of any of claims 1-12 comprising culturing a host cell which has been transformed so that it contains DNA coding for the mutant protein, the DNA coding for the mutant protein being operatively linked to expression control sequenced, the culturing taking place under conditions permitting expression of the mutant protein.

17. An *in vitro* method of binding aggregated immunoglobulin or immune complexes contained in a fluid comprising contacting the aggregated immunoglobulin or immune complexes with the mutant protein of any one of claims 1-12.

18. An *in vitro* method of removing aggregated immunoglobulin or immune complexes from a fluid comprising contacting the aggregated immunoglobulin or immune complexes with the mutant protein of any one of claims 1-12.

19. An *in vitro* method of quantitating immune complexes comprising contacting the immune complexes with the mutant protein of any one of claims 1-12.

20. A device for removing aggregated immunoglobulin or immune complexes from a fluid comprising:
the mutant protein of any one of claims 1-12 bound to a solid surface; and
a means for encasing the solid surface so that the fluid can be contacted with the solid surface.

21. A kit for quantitating immune complexes comprising a container of the mutant protein of any one of claims 1-12.

22. A pharmaceutical composition comprising the mutant protein of any one of claims 1-12 and a pharmaceutically-acceptable carrier.

23. Use of a mutant protein according to any of claims 1-12 in the preparation of a medicament for reducing the level of immune complexes in a mammal.

24. Use of a mutant protein according to any of claims 1-12 in the preparation of a medicament for treating a viral infection in a mammal.

25. Use of a mutant protein according to any of claims 1-12 in the preparation of a medicament for treating a bacterial infection in a mammal suffering therefrom.

26. Use of a mutant protein according to any of claims 1-12 in the preparation of a medicament for treating endotoxic shock in a mammal.

27. Use of a mutant protein according to any of claims 1-12 in the preparation of a medicament for treating cancer in a mammal.

28. The mutant protein of any one of claims 1-12 which is labelled.

## Patentansprüche

1. Mutiertes Protein, welches die gleiche Aminosäuresequenz wie die Aminosäuresequenz einer nicht-mutierten Untereinheit von C-reaktivem Protein (CRP) oder eines nicht-mutierten präCRP hat, außer daß
(i) mindestens eine Aminosäure der nicht-mutierten CRP-Untereinheit oder des nicht-mutierten präCRP deletiert ist, oder
(ii) mindestens eine Aminosäure der nicht-mutierten CRP-Untereinheit oder des nicht-mutierten präCRP gegen eine andere Aminosäure ausgetauscht ist, oder
(iii) mindestens eine Aminosäure zu der nicht-mutierten CRP-Untereinheit oder dem nicht-mutierten präCRP hinzugefügt ist, oder
(iv) eine Kombination solcher Änderungen durchgeführt worden ist,
wobei die eine oder mehreren hinzugefügten Aminosäuren, die eine oder mehreren deletierten Aminosäuren und die eine oder mehreren ausgetauschten Aminosäuren derart ausgewählt sind, daß das mutierte Protein weniger zur Bildung kovalent vernetzter Aggregate neigt als die nicht-mutierte CRP-Untereinheit oder das nicht-mutierte präCRP,
wobei das mutierte Protein auch mit einem für neo-CRP Antigenizität spezifischen Antikörper reagiert und mindestens eine der biologischen Aktivitäten von modifiziertem CRP aufweist.

2. Mutiertes Protein nach Anspruch 1, wobei mindestens eines der Cysteine der nicht-mutierten CRP-Untereinheit oder des nicht-mutierten präCRP deletiert oder gegen eine andere Aminosäure ausgetauscht ist.

3. Mutiertes Protein nach Anspruch 1, wobei die eine oder mehreren hinzugefügten Aminosäuren, die eine oder mehreren deletierten Aminosäuren und die eine oder mehreren ausgetauschten Aminosäuren weiterhin derart ausgewählt sind, daß das mutierte Protein in wäßrigen Medien besser löslich ist als die nicht-mutierte CRP-Untereinheit oder das nicht-mutierte präCRP.

4. Mutiertes Protein nach Anspruch 1, wobei mindestens eines der Lysine des nicht-mutierten präCRP oder der nicht-mutierten CRP-Untereinheit deletiert oder gegen eine andere Aminosäure ausgetauscht ist.

5. Mutiertes Protein, welches die gleiche Aminosäuresequenz wie die Aminosäuresequenz einer nicht-mutierten Untereinheit von C-reaktivem Protein (CRP) hat, außer daß
(i) mindestens eine Aminosäure der nicht-mutierten CRP-Untereinheit deletiert ist, oder
(ii) mindestens eine Aminosäure der nicht-mutierten CRP-Untereinheit gegen eine andere Aminosäure ausgetauscht ist, oder
(iii) mindestens eine Aminosäure zu der nicht-mutierten CRP-Untereinheit hinzugefügt ist, oder
(iv) eine Kombination solcher Änderungen durchgeführt worden ist,
wobei die eine oder mehreren hinzugefügten Aminosäuren, die eine oder mehreren deletierten Aminosäuren und die eine oder mehreren ausgetauschten Aminosäuren derart ausgewählt sind, daß das mutierte Protein weniger zur Bildung kovalent vernetzter Aggregate neigt als die nicht-mutierte CRP-Untereinheit,
wobei das mutierte Protein auch mit einem für neo-CRP Antigenizität spezifischen Antikörper reagiert und mindestens eine der biologischen Aktivitäten von modifiziertem CRP aufweist.

6. Mutiertes Protein nach Anspruch 5, wobei mindestens eines der Cysteine der nicht-mutierten CRP-Untereinheit deletiert oder gegen eine andere Aminosäure ausgetauscht ist.

7. Mutiertes Protein nach Anspruch 6, wobei mindestens eines der Cysteine der nicht-mutierten CRP-Untereinheit gegen eine Aminosäure, ausgewählt aus der Gruppe, bestehend aus Alanin, Glycin, Valin, Leucin, Isoleucin, Serin, Methionin und Threonin, ausgetauscht ist.

8. Mutiertes Protein nach Anspruch 7, wobei mindestens eines der Cysteine der nicht-mutierten CRP-Untereinheit gegen ein Alanin ausgetauscht ist.

9. Mutiertes Protein nach Anspruch 6, wobei alle Cysteine der nicht-mutierten CRP-Untereinheit deletiert oder gegen eine andere Aminosäure ausgetauscht sind.

10. Mutiertes Protein nach Anspruch 9, wobei alle Cysteine der nicht-mutierten CRP-Untereinheit gegen eine Aminosäure, ausgewählt aus der Gruppe, bestehend aus Alanin, Glycin, Valin, Leucin, Isoleucin, Serin, Methionin und Threonin, ausgetauscht sind.

11. Mutiertes Protein nach Anspruch 10, wobei alle Cysteine der nicht-mutierten CRP-Untereinheit gegen Alanine ausgetauscht sind.

12. Mutiertes Protein nach Anspruch 5, wobei mindestens eines der Lysine der nicht-mutierten CRP-Untereinheit deletiert oder gegen eine andere Aminosäure ausgetauscht ist.

13. DNA-Molekül, kodierend für das mutierte Protein nach einem der Ansprüche 1 bis 12.

14. Vektor zur Expression des mutierten Proteins nach einem der Ansprüche 1 bis 12, umfassend für das mutierte Protein kodierende DNA, operativ mit Expressionskontrollsequenzen verknüpft.

15. Wirtszelle, die transformiert ist, so daß sie DNA enthält, welche für das mutierte Protein nach einem der Ansprüche 1 bis 12 kodiert, wobei die für das mutierte Protein kodierende DNA operativ mit Expressionskontrollsequenzen verknüpft ist.

16. Verfahren zur Herstellung des mutierten Proteins nach einem der Ansprüche 1 bis 12, umfassend das Kultivieren einer Wirtszelle, die transformiert ist, so daß sie DNA enthält, welche für das mutierte Protein kodiert, wobei die für das mutierte Protein kodierende DNA operativ mit Expressionskontrollsequenzen verknüpft ist, wobei das Kultivieren unter Bedingungen erfolgt, welche eine Expression des mutierten Proteins gestatten.

17. In vitro Verfahren zur Bindung von aggregiertem Immunglobulin oder Immunkomplexen, das bzw. die in einem Fluid enthalten sind, umfassend das in Kontakt Bringen des aggregierten Immunglobulins oder der Immunkomplexe mit dem mutierten Protein nach einem der Ansprüche 1 bis 12.

18. In vitro Verfahren zur Entfernung von aggregiertem Immunglobulin oder Immunkomplexen aus einem Fluid, umfassend das in Kontakt Bringen des aggregierten Immunglobulins oder der Immunkomplexe mit dem mutierten Protein nach einem der Ansprüche 1 bis 12.

19. In vitro Verfahren zur Quantifizierung von Immunkomplexen, umfassend das in Kontakt Bringen der Immunkomplexe mit dem mutierten Protein nach einem der Ansprüche 1 bis 12.

20. Vorrichtung zur Entfernung von aggregiertem Immunglobulin oder Immunkomplexen aus einem Fluid, umfassend:
das mutierte Protein nach einem der Ansprüche 1 bis 12, an eine Festoberfläche gebunden, und
ein Gehäusemittel für die Festoberfläche, so daß das Fluid mit der Festoberfläche in Kontakt gebracht werden kann.

21. Kit zur Quantifizierung von Immunkomplexen, umfassend einen Behälter des mutierten Proteins nach einem der Ansprüche 1 bis 12.

22. Pharmazeutische Zusammensetzung, umfassend das mutierte Protein nach einem der Ansprüche 1 bis 12 und einen pharmazeutisch akzeptablen Träger.

23. Verwendung eines mutierten Proteins nach einem der Ansprüche 1 bis 12 zur Herstellung eines Arzneimittels zur Verringerung des Gehalts von Immunkomplexen in einem Säuger.

24. Verwendung eines mutierten Proteins nach einem der Ansprüche 1 bis 12 zur Herstellung eines Arzneimittels zur Behandlung einer viralen Infektion in einem Säuger.

25. Verwendung eines mutierten Proteins nach einem der Ansprüche 1 bis 12 zur Herstellung eines Arzneimittels zur Behandlung einer bakteriellen Infektion in einem daran erkrankten Säuger.

26. Verwendung eines mutierten Proteins nach einem der Ansprüche 1 bis 12 zur Herstellung eines Arzneimittels zur Behandlung von endotoxischem Schock in einem Säuger.

27. Verwendung eines mutierten Proteins nach einem der Ansprüche 1 bis 12 zur Herstellung eines Arzneimittels zur Behandlung von Krebs in einem Säuger.

28. Mutiertes Protein nach einem der Ansprüche 1 bis 12, das markiert ist.

## Revendications

1. Protéine mutante qui a une séquence d'aminoacides identique à la séquence d'aminoacides d'une sous-unité de protéine C-réactive (CRP) non mutée ou d'une préCRP non mutée, sauf que
(i) au moins un aminoacide de la sous-unité de CRP non mutée ou de la préCRP non mutée a été éliminé par délétion, ou
(ii) au moins un aminoacide de la sous-unité de CRP non mutée ou de la préCRP non mutée a été remplacé par un autre aminoacide, ou
(iii) au moins un aminoacide a été ajouté à la sous-unité de CRP non mutée ou à la préCRP non mutée, ou
(iv) une association de ces modifications a été réalisée,
le ou les aminoacides ajoutés, le ou les aminoacides éliminés par délétion et le ou les aminoacides de remplacement étant choisis de telle sorte que la protéine mutante soit moins susceptible de former des agrégats réticulés par covalence que la sous-unité de CRP non mutée ou la préCRP non mutée,
la protéine mutante réagissant également avec un anticorps spécifique de l'antigénicité de néo-CRP et présentant au moins une des activités biologiques de la CRP modifiée.

2. Protéine mutante suivant la revendication 1, dans laquelle au moins une des cystéines de la sous-unité de CRP non mutée ou de la pré-CRP non mutée a été éliminée par délétion ou remplacée par un autre aminoacide.

3. Protéine mutante suivant la revendication 1, dans laquelle le ou les aminoacides ajoutés, le ou les aminoacides éliminés par délétion et le ou les aminoacides de remplacement sont également choisis de telle sorte que la protéine mutante soit plus soluble dans des milieux aqueux que la sous-unité de CRP non mutée ou la préCRP non mutée.

4. Protéine mutante suivant la revendication 1, dans laquelle au moins une des lysines de la préCRP non mutée ou de la sous-unité de CRP non mutée a été éliminée par délétion ou remplacée par un autre aminoacide.

5. Protéine mutante qui a une séquence d'aminoacides identique à la séquence d'aminoacides d'une sous-unité de protéine C-réactive (CRP) non mutée, sauf que
(i) au moins un aminoacide de la sous-unité de CRP non mutée a été éliminé par délétion, ou
(ii) au moins un aminoacide de la sous-unité de CRP non mutée a été remplacé par un autre aminoacide, ou
(iii) au moins un aminoacide a été ajouté à la sous-unité de CRP non mutée, ou bien
(iv) une association de ces modifications a été réalisée,
le ou les aminoacides ajoutés, le ou les aminoacides éliminés par délétion et le ou les aminoacides de remplacement étant choisis de telle sorte que la protéine mutante soit moins susceptible de former des agrégats réticulés par covalence que la sous-unité de CRP non mutée,
la protéine mutante réagissant également avec un anticorps spécifique de l'antigénicité de néo-CRP et présentant au moins une des activités biologiques de la CRP modifiée.

6. Protéine mutante suivant la revendication 5, dans laquelle au moins une des cystéines de la sous-unité de CRP non mutée a été éliminée par délétion ou remplacée par un autre aminoacide.

7. Protéine mutante suivant la revendication 6, dans laquelle au moins une des cystéines de la sous-unité de CRP non mutée a été remplacée par un aminoacide choisi dans le groupe consistant en l'alanine, la glycine, la valine, la leucine, l'isoleucine, la sérine, la méthionine et la thréonine.

8. Protéine mutante suivant la revendication 7, dans laquelle au moins une des cystéines de la sous-unité de CRP non mutée a été remplacée par une alanine.

9. Protéine mutante suivant la revendication 6, dans laquelle toutes les cystéines de la sous-unité de CRP non mutée ont été éliminées par délétion ou remplacées par un autre aminoacide.

10. Protéine mutante suivant la revendication 9, dans laquelle toutes les cystéines de la sous-unité de CRP non mutée ont été remplacées par un aminoacide choisi dans le groupe consistant en l'alanine, la glycine, la valine, la leucine, l'isoleucine, la sérine, la méthionine et la thréonine.

11. Protéine mutante suivant la revendication 10, dans laquelle toutes les cystéines de la sous-unité de CRP non mutée ont été remplacées par des alanines.

12. Protéine mutante suivant la revendication 5, dans laquelle au moins une des lysines de la sous-unité de CRP non mutée a été éliminée par délétion ou remplacée par un autre aminoacide.

13. Molécule d'ADN codant pour la protéine mutante suivant l'une quelconque des revendications 1 à 12.

14. Vecteur pour l'expression de la protéine mutante suivant l'une quelconque des revendications 1 à 12, comprenant l'ADN codant pour la protéine mutante lié de manière fonctionnelle à des séquences de régulation d'expression.

15. Cellule hôte qui a été transformée de telle sorte qu'elle contienne l'ADN codant pour la protéine mutante suivant l'une quelconque des revendications 1 à 12, l'ADN codant pour la protéine mutante étant lié de manière fonctionnelle à des séquences de régulation d'expression.

16. Procédé pour la production de la protéine mutante suivant l'une quelconque des revendications 1 à 12, comprenant la culture d'une cellule hôte qui a été transformée de telle sorte qu'elle contienne l'ADN codant pour la protéine mutante, l'ADN codant pour la protéine mutante étant lié de manière fonctionnelle à des séquences de régulation d'expression, la culture s'effectuant dans des conditions permettant l'expression de la protéine mutante.

17. Procédé in vitro pour la liaison de complexes d'immunoglobulines ou complexes immuns agrégés présents dans un liquide, comprenant la mise en contact des complexes d'immunoglobulines ou complexes immuns agrégés avec la protéine mutante suivant l'une quelconque des revendications 1 à 12.

18. Procédé in vitro pour séparer des complexes d'immunoglobulines ou complexes immuns agrégés d'un liquide, comprenant la mise en contact des complexes d'immunoglobulines ou complexes immuns agrégés avec la protéine mutante suivant l'une quelconque des revendications 1 à 12.

19. Procédé in vitro pour quantifier des complexes immuns, comprenant la mise en contact des complexes immuns avec la protéine mutante suivant l'une quelconque des revendications 1 à 12.

20. Dispositif pour séparer des complexes d'immunoglobulines ou complexes immuns agrégés d'un fluide, comprenant :
la protéine mutante suivant l'une quelconque des revendications 1 à 12 liée à une surface solide ; et
un moyen pour enfermer la surface solide de telle sorte que le liquide puisse être mis en contact avec la surface solide.

21. Kit pour quantifier des complexes immuns, comprenant un récipient contenant la protéine mutante suivant l'une quelconque des revendications 1 à 12.

22. Composition pharmaceutique comprenant la protéine mutante suivant l'une quelconque des revendications 1 à 12 et un support pharmaceutiquement acceptable.

23. Utilisation d'une protéine mutante suivant l'une quelconque des revendications 1 à 12 dans la préparation d'un médicament destiné à réduire le taux de complexes immuns chez un mammifère.

24. Utilisation d'une protéine mutante suivant l'une quelconque des revendications 1 à 12 dans la préparation d'un médicament destiné au traitement d'une infection virale chez un mammifère.

25. Utilisation d'une protéine mutante suivant l'une quelconque des revendications 1 à 12, dans la préparation d'un médicament destiné au traitement d'une infection bactérienne chez un mammifère souffrant de cette infection.

26. Utilisation d'une protéine mutante suivant l'une quelconque des revendications 1 à 12 dans la préparation d'un médicament destiné au traitement d'un choc endotoxique chez un mammifère.

27. Utilisation d'une protéine mutante suivant l'une quelconque des revendications 1 à 12 dans la préparation d'un médicament destiné au traitement d'un cancer chez un mammifère.

28. Protéine mutante suivant l'une quelconque des revendications 1 à 12, qui est marquée.


SEQUENCE LISTING

(1) GENERAL INFORMATION:
(i) APPLICANT:
(A) NAME: Immtech International, Inc.
(B) STREET: 3110, 20 North Wacker Drive
(C) CITY: Chicago
(D) STATE: Illinois
(E) COUNTRY: USA
(F) ZIP: 60606
(ii) TITLE OF INVENTION: A Mutant Protein And Methods
And Materials For Making And Using It
(iii) NUMBER OF SEQUENCES: 10
(iv) COMPUTER READABLE FORM:
(A) MEDIUM TYPE: Diskette
(B) COMPUTER: IBM PC compatible
(C) OPERATING SYSTEM: PC-DOS/MS-DOS
(v) CURRENT APPLICATION DATA:
(A) APPLICATION NUMBER: EP-94909837.0
(B) FILING DATE: 24-FEB-1994

(2) INFORMATION FOR SEQ ID NO:1:
(i) SEQUENCE CHARACTERISTICS:
(A) LENGTH: 28 bases
(B) TYPE: nucleic acid
(C) STRANDEDNESS: single
(D) TOPOLOGY: linear

(xi) SEQUENCE DESCRIPTION:SEQ ID NO:1:
GGGCCAT ATG CAG ACA GAC ATG TCG AGG 28
Met Gln Thr Asp Met Ser Arg
                5

(2) INFORMATION FOR SEQ ID NO:2:
(i) SEQUENCE CHARACTERISTICS:
(A) LENGTH: 20 bases
(B) TYPE: nucleic acid
(C) STRANDEDNESS: single
(D) TOPOLOGY: linear

(xi) SEQUENCE DESCRIPTION:SEQ ID NO:2:
GAAGCGCCAC AGTGAAGGCT 20

(2) INFORMATION FOR SEQ ID NO:3:
(i) SEQUENCE CHARACTERISTICS:
(A) LENGTH: 16 bases

(B) TYPE: nucleic acid
(C) STRANDEDNESS: single
(D) TOPOLOGY: linear

(xi) SEQUENCE DESCRIPTION:SEQ ID NO:3:
C ACT GTG GCG CTC CAC 16
Thr Val Ala Leu His
                5

(2) INFORMATION FOR SEQ ID NO:4:
(i) SEQUENCE CHARACTERISTICS:
(A) LENGTH: 19 bases
(B) TYPE: nucleic acid
(C) STRANDEDNESS: single
(D) TOPOLOGY: linear

(xi) SEQUENCE DESCRIPTION:SEQ ID NO:4:
TTGTCGCGAT GTGTACTGG 19

(2) INFORMATION FOR SEQ ID NO:5:
(i) SEQUENCE CHARACTERISTICS:
(A) LENGTH: 17 bases
(B) TYPE: nucleic acid
(C) STRANDEDNESS: single
(D) TOPOLOGY: linear

(xi) SEQUENCE DESCRIPTION:SEQ ID NO:5:
CAC ATC GCG ACA AGC TG 17
His Ile Ala Thr Ser
                5

(2) INFORMATION FOR SEQ ID NO:6:
(i) SEQUENCE CHARACTERISTICS:
(A) LENGTH: 24 bases
(B) TYPE: nucleic acid
(C) STRANDEDNESS: single
(D) TOPOLOGY: linear

(xi) SEQUENCE DESCRIPTION:SEQ ID NO:6:
GGCGAGATCT GAGGTACCTT CAGG 24

(2) INFORMATION FOR SEQ ID NO:7:
(i) SEQUENCE CHARACTERISTICS:
(A) LENGTH: 12 bases
(B) TYPE: nucleic acid
(C) STRANDEDNESS: double
(D) TOPOLOGY: circular

(xi) SEQUENCE DESCRIPTION:SEQ ID NO:7:
TTT GGC CAG ACA 12
Phe Gly Gln Thr

(2) INFORMATION FOR SEQ ID NO:8:
(i) SEQUENCE CHARACTERISTICS:
(A) LENGTH: 15 bases
(B) TYPE: nucleic acid
(C) STRANDEDNESS: double
(D) TOPOLOGY: circular

(xi) SEQUENCE DESCRIPTION:SEQ ID NO:8:
CAT ATG GCT AGC ATG 15
Met Ala Ser Met

(2) INFORMATION FOR SEQ ID NO:9:
(i) SEQUENCE CHARACTERISTICS:
(A) LENGTH: 21 bases
(B) TYPE: nucleic acid
(C) STRANDEDNESS: double
(D) TOPOLOGY: circular

(xi) SEQUENCE DESCRIPTION:SEQ ID NO:9:
GCT TTT GGC CAG ACA GAC ATG 21
Ala Phe Gly Gln Thr Asp Met
                5

(2) INFORMATION FOR SEQ ID NO:10:
(i) SEQUENCE CHARACTERISTICS:
(A) LENGTH: 24 bases
(B) TYPE: nucleic acid
(C) STRANDEDNESS: double
(D) TOPOLOGY: circular

(xi) SEQUENCE DESCRIPTION:SEQ ID NO:10:
CAT ATG GCT AGC CAG ACA GAC ATG 24
Met Ala Ser Gln Thr Asp Met
                5